# EUROPÄISCHE PATENTSCHRIFT

(11) **EP 0 655 995 B1**
(45) Veröffentlichungstag und Bekanntmachung des Hinweises auf die Patenterteilung: **06.05.1998**
(21) Anmeldenummer: 93917789.5
(22) Anmeldetag: 13.08.1993
(51) Int. Cl.: C07C 251/42, C07C 251/52, C07C 251/54, A01N 35/10, A01N 43/16, A01N 43/18, C07D 309/06, C07D 335/02

(54) **CYCLOHEXENONOXIMETHER, IHRE HERSTELLUNG UND IHRE VERWENDUNG**
CYCLOHEXENONOXIME ETHERS, THEIR PREPARATION AND THEIR USE
ETHERS DE CYCLOHEXENONOXIME, LEUR PREPARATION ET LEUR UTILISATION

(30) Priorität: 22.08.1992 DE 4227896
(43) Veröffentlichungstag der Anmeldung: 07.06.1995
(73) Patentinhaber: BASF Aktiengesellschaft, 67063 Ludwigshafen (DE)
(72) Erfinder: MISSLITZ, Ulf, D-6730 Neustadt (DE); MEYER, Norbert, D-6802 Ladenburg (DE); KAST, Juergen, D-6737 Boehl-Iggelheim (DE); KOLASSA, Dieter, D-6703 Limburgerhof (DE); WALTER, Helmut, D-6719 Obrigheim (DE); WESTPHALEN, Karl-Otto, D-6720 Speyer (DE); GERBER, Matthias, D-6703 Limburgerhof (DE); KARDORFF, Uwe, D-6800 Mannheim 1 (DE)
(86) Internationale Anmeldenummer: EP9302158
(87) Internationale Veröffentlichungsnummer: WO9404489

(56) Entgegenhaltungen:
- EP-A- 0 157 184
- EP-A- 0 230 235
- EP-A- 0 310 186
- EP-A- 0 387 568
- EP-A- 0 456 112
- CHEMICAL ABSTRACTS, vol. 108, no. 23, 6. Juni 1988, Columbus, Ohio, US; abstract no. 203355r, T. GOMYO ET. AL. 'Residue analysis of herbicide sethoxydim and its metabolites in crops.' Seite 562 ;Spalte 1 ; & NIPPON NOYAKU GAKKASHI Bd. 12, Nr. 4 , 1987 Seiten 729 - 37
- CHEMICAL ABSTRACTS, vol. 110, no. 17, 24. April 1989, Columbus, Ohio, US; abstract no. 149474e, R. HUBER ET. AL. 'The metabolism of cycloxydim in soybeans' Seite 259 ;Spalte 2 ; & BRIGHTON CROP PROT. CONF. - PESTS DIS. Nr. 1 , 1988 Seiten 335 - 41
- Proceedings 1985 British Crop Protection Conference, Weeds, vol. 1, Seiten 93-8.

## Beschreibung

Die vorliegende Erfindung betrifft Cyclohexenonoximether der Formel I 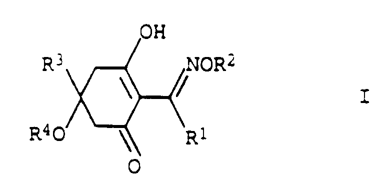 in der die Substituenten die folgende Bedeutung haben:
- R¹: C₁-C₆-Alkyl;
- R²: C₁-C₁₀-Alkyl, C₁-C₁₀-Halogenalkyl, C₃-C₁₀-Alkenyl, C₃-C₁₀-Halogenalkenyl, C₃-C₁₀-Alkinyl oder C₃-C₁₀-Halogenalkinyl; -A¹-O-N=CH-Ph oder -A²-W, wobei
A¹ für C₂-C₄-Alkylen steht, welches ein bis drei C₁-C₃-Alkylgruppen tragen kann;
Ph für Phenyl steht, welches unsubstituiert sein oder ein bis drei Reste tragen kann, ausgewählt aus einer Gruppe bestehend aus Nitro, Cyano, Halogen, C₁-C₄-Alkyl und C₁-C₄-Halogenalkyl;
A² für C₁-C₆-Alkylen, C₃-C₆-Alkenylen oder C₃-C₆-Alkinylen steht, wobei diese Reste jeweils ein bis drei der folgenden Gruppen tragen können: Halogen und/oder C₁-C₃-Alkyl; oder für C₂-C₅-Alkylenoxy, C₂-C₅-Alkenylenoxy oder C₂-C₄-Alkylenoxy-C₁-C₃-alkylen mit insgesamt drei bis fünf Kohlenstoffatomen steht, wobei diese Reste jeweils ein bis drei C₁-C₃-Alkylgruppen tragen können;
W für Phenyl, Pyridyl oder Thienyl steht, wobei die Ringe jeweils unsubstituiert sein oder ein bis drei der folgenden Reste tragen konnen: Nitro, Cyano, Halogen, C₁-C₄-Alkyl und/oder C₁-C₄-Halogenalkyl;
- R³: C₁-C₆-Alkyl, welches eine der folgenden Gruppen trägt: C₁-C₄-Alkoxy oder C₁-C₄-Alkylthio; C₃-C₇-Cycloalkyl oder C₅-C₇-Cycloalkenyl, wobei diese Reste unsubstituiert sein oder ein bis drei der folgenden Substituenten tragen konnen: Hydroxy, Halogen, C₁-C₄-Alkyl C₁-C₄-Halogenalkyl, C₁-C₄-Alkoxy und/oder C₁-C₄-Alkylthio;
ein 5-gliedriger, gesättigter Heterocyclus, welcher neben Kohlenstoffringgliedern ein oder zwei Sauerstoff- und/oder Schwefelatome enthalten kann, wobei dieser Ring unsubstituiert sein oder ein bis drei der folgenden Substituenten tragen kann: C₁-C₄-Alkyl, C₁-C₄-Halogenalkyl, C₁-C₄-Alkoxy und/oder C₁-C₄-Alkylthio; ein 6- oder 7-gliedriger, gesattigter oder ein- oder zweifach ungesättigter Heterocyclus, welcher neben Kohlenstoffringgliedern ein oder zwei Sauerstoff- und/oder Schwefelatome enthalten kann, wobei dieser Ring unsubstituiert sein oder ein bis drei der folgenden Substituenten tragen kann: Hydroxy, Halogen, C₁-C₄-Alkyl, C₁-C₄-Halogenalkyl, C₁-C₄-Alkoxy und/oder C₁-C₄-Alkylthio; ein 5-gliedriger Heteroaromat, welcher neben Kohlenstoffringgliedern ein oder zwei Stickstoffatome und ein Sauerstoff- oder ein Schwefelatom enthält, wobei dieser Ring unsubstituiert sein oder ein bis drei der folgenden Substituenten tragen kann: Cyano, Halogen, C₁-C₄-Alkyl, C₁-C₄-Halogenalkyl, C₁-C₄-Alkoxy, C₁-C₄-Alkylthio, C₂-C₆-Alkenyl, C₂-C₆-Alkinyl und/oder C₁-C₄-Alkoxy-C₁-C₄-alkyl;
Phenyl oder Pyridyl, wobei diese Ringe unsubstituiert sein oder ein bis drei der folgenden Substituenten tragen können: Nitro, Cyano, Halogen, C₁-C₄-Alkyl, C₁-C₄-Halogenalkyl, C₁-C₄-Alkoxy, C₁-C₄-Alkylthio, C₃-C₆-Alkenyloxy, C₃-C₆-Alkinyloxy und/oder NR^{a}R^{b}, wobei
- R^{a}: für Wasserstoff, C₁-C₄-Alkyl, C₃-C₆-Alkenyl oder C₃-C₆-Alkinyl steht und
- R^{b}: für einen der Reste R^{a} oder für C₁-C₆-Alkylcarbonyl oder Benzoyl steht, wobei der Phenylring seinerseits ein bis drei der folgenden Reste tragen kann: Nitro, Cyano, Halogen, C₁-C₄-Alkyl, C₁-C₄-Halogenalkyl, C₁-C₄-Alkoxy und/oder C₁-C₄-Alkylthio;
- R⁴: Wasserstoff;
C₁-C₁₀-Alkyl, C₁-C₁₀-Halogenalkyl, C₃-C₁₀-Alkenyl, C₃-C₁₀-Halogenalkenyl, C₃-C₁₀-Alkinyl, C₃-C₁₀-Halogenalkinyl, C₁-C₁₀-Alkylcarbonyl oder C₁-C₁₀-Halogenalkylcarbonyl;
Benzoyl, wobei der Phenylring unsubstituiert sein oder ein bis drei der folgenden Reste tragen kann: Nitro, Cyano, Halogen, C₁-C₄-Alkyl, C₁-C₄-Halogenalkyl, C₁-C₄-Alkoxy und/oder C₁-C₄-Alkylthio;
-S(=O)₂-R^{c}; -P(=O)(OR^{d})(OR^{e}) oder -SiR^{f}R^{g}R^{h}, wobei
R^{c}, R^{d}, R^{e}, R^{f}, R^{g} und R^{h} unabhängig voneinander für C₁-C₁₀-Alkyl, C₁-C₁₀-Halogenalkyl oder Phenyl stehen, wobei der Phenylring seinerseits ein bis drei der folgenden Reste tragen kann: Nitro, Cyano, Halogen, C₁-C₄-Alkyl, C₁-C₄-Halogenalkyl, C₁-C₄-Alkoxy und/oder C₁-C₄-Alkylthio;
wobei R³ nicht für 2-(Ethylthio)propyl steht, wenn R¹ Ethyl, R² 3-Chlor-2-propenyl und R⁴ Wasserstoff bedeuten,
sowie die landwirtschaftlich brauchbaren Salze von I und die Ester aus C₁-C₁₀-Carbonsäuren oder anorganischen Säuren und den Verbindungen I.

Außerdem betrifft die Erfindung Verfahren und Zwischenprodukte zur Herstellung dieser Cyclohexenonoximether sowie sie enthaltende Mittel und deren Verwendung zur Bekämpfung von unerwünschtem Pflanzenwuchs.

In der Literatur [Nippon Noyaku Gakkaishi 12, 729 (1987); Weed Sci. 34, 745 (1986); Brighton Crop Prot. Conf.-Pests Dis. 1, 335 (1988)] werden die in der folgenden Tabelle zusammengestellten Verbindungen vom Typ der Cyclohexenonoximether I als Abbauprodukte bekannter Cyclohexenon-Herbizide beschrieben:

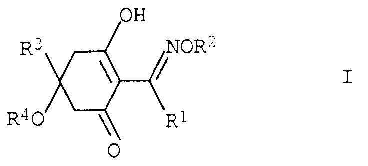

| R¹ | R² | R³ | R⁴ |
|---|---|---|---|
| CH₂CH₂CH₃ | CH₂CH₃ | CH(CH₃)-SO-CH₂CH₃ | H |
| CH₂CH₂CH₃ | CH₂CH₃ | CH(CH₃)-SO₂-CH₂CH₃ | H |
| CH₂CH₂CH₃ | CH₂CH₃ | Tetrahydrothiopyran-3-yl-S-oxid | H |
| CH₂CH₂CH₃ | CH₂CH₃ | Tetrahydrothiopyran-3-yl-S,S-dioxid | H |
| CH₂CH₃ | CH₂CH=CHCl | CH(CH₃)-S-CH₂CH₃ | H |
| CH₂CH₃ | CH₂CH=CHCl | CH(CH₃)-SO-CH₂CH₃ | H |
| CH₂CH₃ | CH₂CH=CHCl | CH(CH₃)-SO₂-CH₂CH₃ | H |
| CH₂CH₂CH₃ | CH₂CH₃ | CH(CH₃)-SO₂-CH₂CH₃ | CH₃ |
| CH₂CH₃ | CH₂CH=CHCl | CH(CH₃)-SO₂-CH₂CH₃ | CH₃ |

Cyclohexenonoximether, die in 5-Position des Cyclohexenonrings lediglich einen Substituenten tragen, sind aus einer Vielzahl von Schriften als Herbizide bekannt (z.B. EP-A 368,227; EP-A 080,301; EP-A 125,094; EP-A 238,021;

EP-A 243,313; EP-A 456,068; EP-A 456,069; EP-A 456,089; EP-A 456,112; EP-A 456,118; EP-A 066,195; US-A 4,249,937).

Außerdem werden Cyclohexenonoximether, die in 5-Position des Cyclohexenonrings zwei Substituenten tragen, von denen einer Hydroxyl bedeutet, in allgemeiner Form von den folgenden Schriften umfaßt: EP-A 385,084; EP-A 380,985; EP-A 387,568; EP-A 387,582; EP-A 459,140; EP-A 430,004; EP-A 464,542. Beispiele für derartige Verbindungen und deren Wirkung sind diesen Druckschriften jedoch nicht zu entnehmen.

Gegenstand der EP-A 136 647 sind schließlich Cyclohexenonderivate der Formel 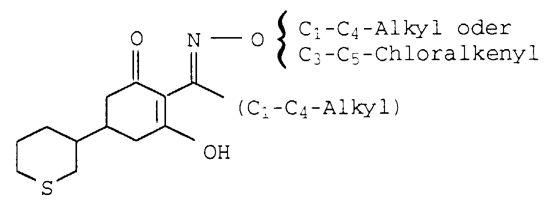 sowie deren Herstellung aus 2-(C₁-C₄-Alkylcarbonyl)-5-(tetrahydrothiopyran-3-yl)-cyclohexan-1,3-dionen.

Aufgabe der vorliegenden Erfindung war es, neue herbizide Wirkstoffe mit verbesserten Eigenschaften bereitzustellen.

Demgemäß wurden die eingangs definierten Cyclohexenonoximether I gefunden. Des weiteren wurden Verfahren und Zwischenprodukte zu ihrer Herstellung und ihre Verwendung in herbiziden Mitteln zur Bekämpfung von unerwünschtem Pflanzenwuchs gefunden.

Die neuen Cyclohexenonoximether I sind auf verschiedene, in der eingangs zitierten Literatur beschriebene, Weise erhältlich.

Besonders vorteilhaft erhält man die Verbindungen der Formel Ia (Cyclohexenonoximether der Formel I, in denen R⁴ Wasserstoff bedeutet) dadurch, daß man ein Triketon der Formel II in an sich bekannter Weise in einem inerten organischen Losungsmittel in Gegenwart einer Base mit einem Säurehalogenid der Formel III zu einem Cyclohexenonketon der Formel IV cyclisiert und IV anschließend in an sich bekannter Weise in einem inerten organischen Lösungsmittel mit einem Hydroxylamin der Formel V oder einem entsprechenden Hydroxylammoniumsalz umsetzt: 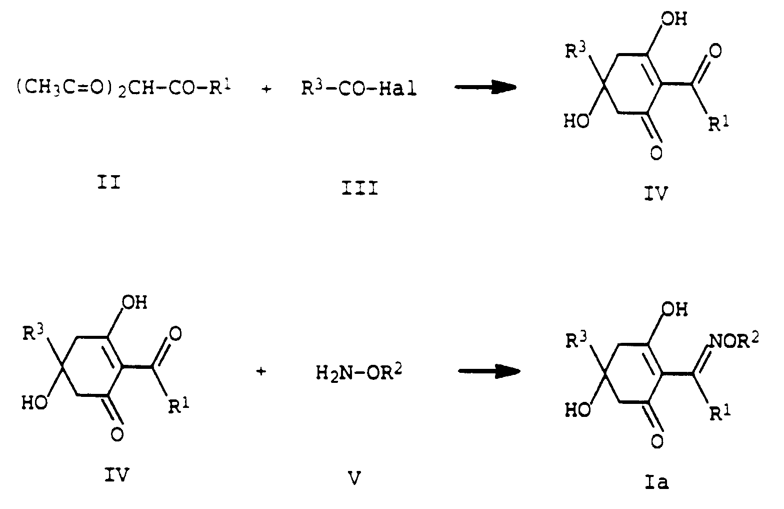 Hal in Formel III steht für ein Halogenatom wie Fluor, Chlor, Brom und Jod, vorzugsweise Chlor und Brom.

Die Umsetzungen werden im einzelnen wie folgt durchgeführt:

Die Herstellung des Cyclohexenonketons der Formel IV erfolgt in an sich bekannter Weise (Angew. Chem. 101, 484 (1989); Chem. Ber. 124, 1845 (1991)) bei Temperaturen von (- 105)°C bis 25°C, vorzugsweise (- 78)°C bis 0°C in einem inerten organischen Lösungsmittel in Gegenwart einer Base.

Geeignete Lösungsmittel sind aliphatische und aromatische Kohlenwasserstoffe wie Pentan, Hexan, Cyclohexan, Petrolether, Toluol, sowie Ether wie Diethylether, Ethylenglycoldimethylether, Diethylenglycoldimethylether, Dioxan und Tetrahydrofuran; besonders bevorzugt ist Tetrahydrofuran. Es konnen aber auch Gemische der genannten Lösungsmittel verwendet werden.

Als Basen Kommen allgemein Hydride, Amide, Alkoholate und Alkylide von Alkalimetall- und Erdalkalimetallionen in Betracht. Beispiele für geeignete Basen sind Hydride wie Lithiumhydrid, Natriumhydrid, Kaliumhydrid und Calziumhydrid, Alkalimetallamide wie Lithiumamid, Natriumamid und Kaliumamid, Dialkylamide des Lithiums oder Natriums wie N,N-Dimethylamid, N,N-Diethylamid, N,N-Diisopropylamid und 2,2,6,6-Tetramethylpiperid, außerdem Alkalimetallalkyle wie Methyllithium, Butyllithium und Phenyllithium, sowie Alkalimetall- und Erdalkalimetallalkoholate wie Natriummethanolat, Natriumethanolat, Kaliumethanolat, Kalium-tert.-butanolat und Magnesiummethanolat, vorzugsweise Lithium-N,N-diisopropylamid und Lithium-N,N-(bistrimethylsilyl)amid.

Die Basen werden im allgemeinen in äquimolaren Mengen verwendet. Dementsprechend setzt man üblicherweise ca. 3 mol-Äq. der Base pro mol Triketon II ein (entsprechend 1 mol-Aq. Base pro Ketofunktion). Es kann vorteilhaft sein, die Base in einem Überschuß, von beispieisweise bis ca. 30 mol-% einzusetzen.

Nach bisherigen Erkenntnissen verläuft diese Umsetzung vorteilhaft in Gegenwart eines Lösungsvermittlers oder eines Hilfsreagenses. Als in dieser Hinsicht besonders geeignet haben sich N,N,N',N'-Tetramethylethylendiamin, Hexamethylphosphorsäuretriamid, N,N'-Dimethylpropylenharnstoff und N,N'-Dimethylethylenharnstoff erwiesen.

Das Hilfsreagens wird im allgemeinen in etwa äquimolaren Mengen bezogen auf die Menge an eingesetzter Base verwendet.

Die Edukte werden im allgemeinen in äquimolaren Mengen miteinander umgesetzt. Es kann für die Ausbeute vorteilhaft sein, das Säurehalogenid III in einem Überschuß bezogen auf das Triketon II einzusetzen.

Üblicherweise geht man bei der Umsetzung so vor, daß man zunachst das Triketon II mit der Base enolisiert und anschließend das Säurehalogenid zugibt.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach braunlicher, zaher Ole an, die unter vermindertem Druck und bei maßig erhohter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Verbindungen IV benotigten Ausgangsstoffe II und III sind in der Literatur bekannt (JP-A 49/034,650; US-A 3,947,488; Organikum, 17. Auflage, S. 423 f, VEB Deutscher "Verlag der Wissenschaften, Berlin 1988) oder konnen gemäß der zitierten Literatur hergestellt werden.

Die Umsetzung des Cyclohexenonketons der Formel IV mit einem Hydroxylamin der Formel V bzw. einem entsprechenden Ammoniumsalz erfolgt in an sich bekannter Weise (gemäß der eingangs zitierten Literatur) bei Temperaturen von 0°C bis zum Siedepunkt des verwendeten Lösungsmittels, vorzugsweise 20°C bis 80°C in einem inerten organischen Lösungsmittel. Sofern man das Hydroxylamin in Form seines Salzes verwendet, erfordert die Umsetzung zusatzlich die Gegenwart einer Base.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform, 1,2-Dichlorethan und Chlorbenzol, Alkohole wie Methanol, Ethanol, n-Propanol und Iso-propanol, Ether wie Diethylether, Diisopropylether, tert.-Butylmethylether, Ethylenglycoldimethylether, Diethylenglycoldimethylether, Dioxan und Tetrahydrofuran, organische Ester wie Essigsäureethylester, sowie Dimethylsulfoxid, besonders bevorzugt sind Toluol und Methanol. Es können aber auch Gemische der genannten Lösungsmittel verwendet werden.

Bei der Umsetzung der Ammoniumsalze finden im allgemeinen Basen wie Carbonate, Hydrogencarbonate, Acetate, Alkoholate, Hydroxide oder Oxide von Alkalimetall- und Erdalkalimetallionen Anwendung. Beispiele für geeignete Basen sind Alkalimetall- und Erdalkalimetallcarbonate wie Lithiumcarbonat und Calziumcarbonat, Alkalimetallhydrogencarbonate wie Natriumhydrogencarbonat, Alkalimetallacetate wie Natriumacetat, Alkalimetall- und Erdalkalimetallalkoholate wie Natriummethanolat, Natriumethanolat, Kaliumethanolat, Kalium-tert.-butanolat und Dimethoxymagnesium, Alkalimetall- und Erdalkalimetallhydroxide wie Lithiumhydroxid, Natriumhydroxid, Kaliumhydroxid, Calziumhydroxid, sowie Alkalimetall- und Erdalkalimetalloxide wie Lithiumoxid, Natriumoxid, Calziumoxid und Magnesiumoxid.

Besonders bevorzugt wird die Umsetzung von Ammoniumsaizen der Verbindungen V mit IV in Methanol als Lösungsmittel und gegebenenfalls mit Natriumhydrogencarbonat als Base durchgeführt.

Die Basen werden im allgemeinen in Mengen von 0,5 bis 2 mol-Aq. bezogen auf die Menge an Ammoniumsalz eingesetzt.

Sofern für die Umsetzung nicht die Ammoniumsalze sondern die freien Basen V eingesetzt werden, wird das Hydroxylamin üblicherweise in Form seiner wäßrigen Lösung eingesetzt.

Dementsprechend kann die Umsetzung in Abhängigkeit vom organischen Lösungsmittel in einem ein- oder zweiphasigen Lösungsmittelsystem vorgenommen werden.

Für diese Variante eignen sich beispielsweise Alkohole wie Methanol, Ethanol, iso-Propanol und Cyclohexanol, aliphatische und aromatische Kohlenwasserstoffe und Halogenkohlenwasserstoffe wie Hexan, Cyclohexan, Toluol, Methylenchlorid und 1,2-Dichlorethan, Nitrile wie Acetonitril und Ether wie Dioxan und Tetrahydrofuran als Lösungsmittel.

Die Edukte IV und V werden im allgemeinen in äquimolaren Mengen miteinander umgesetzt. Es kann für die Ausbeute vorteilhaft sein, einen der Ausgangsstoffe in einem Überschuß von bis ca. 10 mol-% einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach braunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Verbindungen Ia benötigten Hydroxylamine V und deren Ammoniumsalze sind aus der eingangs zitierten Literatur bekannt oder können nach den dort beschriebenen Verfahren hergestellt werden.

Hydroxylamine der Formel Va

H₂N-O-A¹-O-N=CH-Ph Va

in der A¹ und Ph die vorstehend gegeDene Bedeutung haben, sind neu.

Man erhält sie beispielsweise dadurch, daß man ein Bisaminooxyalkan der Formel VII in an sich bekannter Weise (Angew. Makromol. Chem. 184, 125 (1985); Bioorg. Khim. 11, 1574 (1985); J. Org. Chem. 54, 2351 (1989); Houben-Weyl, Methoden in der organischen Chemie, Bd. E 14b, S. 369) in einem inerten organischen Losungsmittel mit einem Aldehyd der Formel VIII umsetzt.

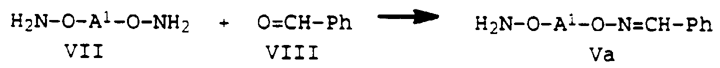

Diese Umsetzung erfolgt üblicherweise bei Temperaturen von 0°C bis 110°C, vorzugsweise 20°C bis 50°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt sind Toluol und Methanol. Es können aber auch Gemische der genannten Lösungsmittel verwendet werden.

Die Edukte werden im allgemeinen in äquimolaren Mengen miteinander umgesetzt. Es kann für die Ausbeute vorteilhaft sein, einen Uberschuß an Bisaminooxyalkan der Formel VII bezogen auf den Aldehyd der Formel VIII einzusetzen, da sich als Nebenprodukte Bis(benzylideniminooxy)alkane der Formel IX bilden können.

Ph-HC=N-O-A¹-O-N=CH-Ph IX

Ausgehend von den Verbindungen der Formel Ia erhält man die Verbindungen der Formel Ib dadurch, daß man daß man eine Verbindung Ia, in an sich bekannter Weise (Organikum, 17. Auflage, S. 198 f. S. 214 f, S. 407 f, VEB Deutscher Verlag der Wissenschaften, Berlin 1988) in einem inerten organischen Lösungsmittel mit einer Verbindung der Formel VI umsetzt.

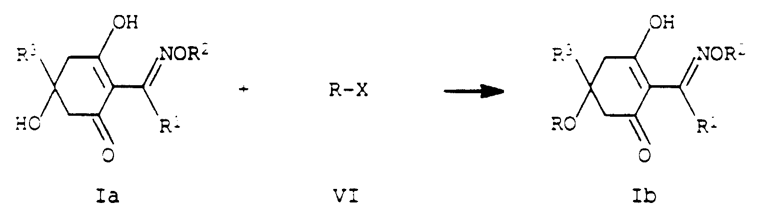

Der Rest R in den Formeln VI und Ib steht für einen der Reste R⁴ gemäß der eingangs gegebenen Definition, ausgenommen Wasserstoff.

X in der Formel VI steht für Halogen wie Fluor, Chlor, Brom und Iod, vorzugsweise Chlor, Brom und Iod.

In Abhängigkeit von der Bedeutung von R in der Formel VI unterscheiden sich die Gruppen X und die Umsetzungsbedingungen:
- A:: Für den Fall, daß R für Alkyl, Alkenyl oder Alkinyl steht, bedeutet X insbesondere Chlor, Brom oder Iod.
Die Umsetzung von Ia mit VI erfolgt in diesem Fall nach den aus der Literatur allgemeinen bekannten Methoden der Veretherung (Lit.: z.B. Organikum, 17. Auflage, S. 198 f, VEB Deutscher Verlag der Wissenschaften, Berlin 1988)
Diese Umsetzung erfolgt üblicherweise bei Temperaturen von 0°C bis 150°C, vorzugsweise 50°C bis 100°C.
Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid und Dimethylformamid, besonders bevorzugt sind Dimethylsulfoxid, Dimethylformamid und Acetonitril.
Es können auch Gemische der genannten Lösungsmittel verwendet werden.
- B:: Für den Fall, daß R für Alkylcarbonyl. Benzoyl, -S(=O)₂-R^{c}, -P(=O)(OR^{d})(OR^{e}) oder -SiR^{f}R^{g}R^{h} steht, bedeutet X insbesondere Chlor.
Die Umsetzung von Ia mit VI erfolgt in diesem Fall nach den aus der Literatur allgemeinen bekannten Methoden der Veresterung (Lit.: z.B. Organikum, 17. Auflage, 5. 407 f., VEB Deutscher Verlag der Wissenschaften, Berlin 1988).
Diese Umsetzung erfolgt üblicherweise bei Temperaturen von (-10)°C bis 100°C, vorzugsweise 0°C bis 50°C.
Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, besonders bevorzugt halogenierte Kohlenwasserstoffe. Es können auch Gemische der genannten Lösungsmittel verwendet werden.

Die Cyclohexenonoximether der Formel I können bei der Herstellung als Isomerengemische anfallen, wobei sowohl E-/Z-Isomerengemische als auch Enantiomeren- oder Diastereomerengemische möglich sind. Diese Gemische können gewünschtenfalls nach den hierfür üblichen Methoden, z.B. durch Chromatographie oder fraktionierte Kristallisation, getrennt werden.

Die allgemeine Formel I steht repräsentativ für alle möglichen tautomeren Formen, in denen die Cyclohexenonoximether I vorliegen können.

Im Hinblick auf die Verwendung der Verbindungen I als Wirkstoffe stehen die eingangs definierten Substituenten für die folgenden Reste. Dementsprechend sind auch die Bezeichnungen der Substituenten in den Vorprodukten IV und Va zu verstehen.
- R¹: steht für C₁-C₆-Alkyl wie Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3, 3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Ethyl und n-Propyl;
- R²: steht für C₁-C₁₀-Alkyl, besonders C₁-C₆-Alkyl wie vorstehend genannt, insbesondere Ethyl;

partiell oder vollständig halogeniertes C₁-C₁₀-Alkyl, vorzugsweise C₁-C₄-Halogenalkyl, insbesondere C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2- Chlor-2-fluorethyl, 2-Chlor-2,2-difluorethyl, 2,2- Dichlor-2-fluorethyl, 2,2,2-Trichlorethyl und Pentafluorethyl, insbesondere 2-Fluorethyl;

C₃-C₁₀-Alkenyl, besonders C₃-C₆-Alkenyl wie 2-Propenyl, 2- Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-l-methyl-2-propenyl und 1-Ethyl-2-methyl-2-propenyl, insbesondere 2-Propenyl und (E)-2-Butenyl;

partiell oder vollständig halogeniertes C₃-C₁₀-Alkenyl wie partiell oder vollstandig durch Halogenatome, insbesondere Fluor und/ oder Chloratome, substituiertes C₃-C₁₀-Alkenyl wie vorstehend im allgemeinen genannt, insbesondere (E)-3-Chlor-2-propenyl;

C₃-C₁₀-Alkinyl, besonders C₃-C₆-Alkinyl wie 2-Propinyl, 2- Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl,1-Ethyl-3-butinyl, 2-Ethyl-3-butinyl und 1-Ethyl-1-methyl-2-propinyl, insbesondere 2-Propinyl;
oder partiell oder vollstandig halogeniertes C₃-C₁₀-Alkinyl, insbesondere durch Fluor und/oder Chlor substituiertes C₃-C₁₀-Alkinyl wie vorstehend im allgemeinen und im besonderen genannt;

-A¹-O-N=CH-Ph oder -A²-W,

wobei
- A¹: für C₂-C₄-Alkylen wie -CH₂CH₂-, -CH₂CH₂CH₂- und -CH₂CH₂CH₂CH₂- steht, welches ein bis drei C₁-C₃-Alkylgruppen wie Methyl, Ethyl, Propyl und 1-Methylethyl, insbesondere Methyl tragen kann;
- Ph: für Phenyl steht, welches ein bis drei der folgenden Gruppen tragen kann:
- Nitro,
- Cyano,
- Halogen wie Fluor, Chlor, Brom und Jod, insbesondere Fluor und Chlor,
- C₁-C₄-Alkyl wie Methyl, Ethyl, n-Propyl, 1-Methylethyl, n-Butyl, 1-Methylpropyl, 2-Methylpropyl und 1,1-Dimethylethyl, insbesondere Methyl, und/oder
- C₁-C₄-Halogenalkyl, wie vorstehend genannt, insbesondere Trifluormethyl;
- A²: für C₁-C₆-Alkylen wie -CH₂-, -CH₂CH₂-, -CH₂CH₂CH₂-, -CH₂CH₂CH₂CH₂-, -CH₂CH₂CH₂CH₂CH₂- und -CH₂CH₂CH₂CH₂CH₂CH₂- steht, oder
für C₃-C₆-Alkenylen wie -CH₂CH=CH-, -CH₂CH=CHCH₂-, -CH₂CH₂CH=CH-, -CH₂CH=CHCH₂CH₂-, -CH₂CH₂CH=CHCH₂-, -CH₂CH₂CH₂CH=CH-, -CH₂CH=CHCH₂CH₂CH₂-, -CH₂CH₂CH=CHCH₂CH₂-, -CH₂CH₂CH₂CH=CHCH₂- und -CH₂CH₂CH₂CH₂CH=CH- steht, oder für C₃-C₆-Alkinylen wie -CH₂C≡C-, -CH₂C≡CCH₂-, -CH₂CH₂C≡C-, -CH₂C≡CCH₂CH₂-, -CH₂CH₂C≡CCH₂-, -CH₂CH₂CH₂C≡C-, -CH₂C≡CCH₂CH₂CH₂-, -CH₂CH₂C≡CCH₂CH₂-, -CH₂CH₂CH₂C≡CCH₂- und -CH₂CH₂CH₂CH₂C≡C- steht,
wobei diese Reste ein bis drei der folgenden Gruppen tragen können:
- Halogen wie vorstehend genannt, insbesondere Fluor und Chlor und/oder
- C₁-C₃-Alkyl wie Methyl, Ethyl, Propyl und 1-Methylethyl, insbesondere Methyl;
oder für C₂-C₅-Alkylenoxy, C₂-C₅-Alkenylenoxy wie -CH₂CH₂O-, -CH₂CH₂CH₂O-, -CH₂CH₂CH₂CH₂O- und -CH₂CH₂CH₂CH₂CH₂O-, oder C₂-C₄-Alkylenoxy-C₁-C₃-alkylen mit insgesamt drei bis fünf Kohlenstoffatomen wie -CH₂CH₂OCH₂-, -CH₂CH₂-OCH₂CH₂-, -CH₂CH₂OCH₂CH₂CH₂-, -CH₂CH₂CH₂OCH₂-, -CH₂CH₂CH₂OCH₂CH₂- und -CH₂CH₂CH₂CH₂OCH₂- steht, wobei diese Reste ein bis drei C₁-C₃-Alkylgruppen wie vorstehend genannt, vorzugsweise Methyl und Ethyl, insbesondere Methyl tragen konnen:
- W: für Phenyl, Pyridyl oder Thienyl steht, wobei diese Reste ein bis drei der folgenden Gruppen tragen können:
- Nitro,
- Cyano,
- Halogen wie vorstehend genannt, insbesondere Fluor oder Chlor,
- C₁-C₄-Alkyl wie vorstehend genannt, insbesondere Methyl und/oder
- C₁-C₄-Halogenalkyl wie vorstehend genannt, insbesondere Trifluormethyl;
- R²: bedeutet besonders bevorzugt C₁-C₁₀-Alkyl, C₁-C₁₀-Halogenalkyl, C₃-C₁₀-Alkenyl, C₃-C₁₀-Halogenalkenyl oder -A²-W;
- R³: steht für
- C₁-C₆-Alkyl wie vorstehend genannt, insbesondere Propyl, welches eine der folgenden Gruppen trägt:
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy, Ethoxy oder C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio, Ethylthio;
   besonders bevorzugt ist C₁-C₄-Alkylthio-C₁-C₆-alkyl;
- C₃-C₇-Cycloalkyl wie Cyclopropyl, Cyclobutyl, Cyclopentyl, Cyclohexyl, Cycloheptyl und Cyclooctyl, insbesondere Cyclopropyl, Cyclohexyl oder
   C₅-C₇-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, insbesondere Cyclohex-3-enyl wobei diese Reste ein bis drei Substituenten tragen können, ausgewählt aus einer Gruppe bestehend aus: Hydroxy,
   Halogen wie vorstehend genannt, insbesondere Fluor oder Chlor,
   C₁-C₄-Alkyl wie vorstehend genannt, insbesondere Methyl C₁-C₄-Halogenalkyl wie vorstehend genannt, insbesondere Trifluormethyl
   C₁-C₄-Alkoxy wie vorstehend genannt, insbesondere Methoxy und/oder
   C₁-C₄-Alkylthio wie vorstehend genannt, insbesondere Methylthio;
   besonders bevorzugt ist C₃-C₇-Cycloalkyl, das eine C₁-C₄-Alkylthiogruppe tragen kann;
- ein 5-gliedriger gesättigter Heterocyclus, welcher neben Kohlenstoffringgliedern ein oder zwei Sauerstoff- und/oder Schwefelatome enthalten kann, wie 2-Tetrahydrofuranyl, 3-Tetrahydrofuranyl, 2-Tetrahydrothienyl, 3-Tetrahydrothienyl, 1,3-Dioxolan-2-yl, 1,3-Dioxolan-4-yl, 1,3-Dithiolan-2-yl, 1,3-Dithiolan-4-yl, 1,3-Thioxolan-2-yl, 1,3-Dioxolan-4-yl und 1,3-Dioxolan-5-yl, wobei dieser Ring ein bis drei Substituenten tragen kann, ausgewählt aus einer Gruppe bestehend aus C₁-C₄-Alkyl wie vorstehend genannt, insbesondere Methyl, C₁-C₄-Halogenalkyl wie vorstehend genannt, insbesondere Trifluormethyl, C₁-C4-Alkoxy wie vorstehend genannt, insbesondere Methoxy und C₁-C₄-Alkylthio wie vorstehend genannt, insbesondere Methylthio;
- ein 6- oder 7-gliedriger, gesättigter oder ein- oder zweifach ungesättigter Heterocyclus, welcher neben Kohlenstoffringgliedern ein oder zwei Sauerstoff- und/oder Schwefelatome enthalten kann, wie Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxepan-5-yl, 1,4-Dioxepan-5-yl, 3,4-Dihydro-2H-pyran-2-yl, 5,6-Dihydro-2H-pyran-2-yl, 2,3-Dihydro-4H-pyran-2-yl, 5,6-Dihydro-4H-pyran-2-yl, 3,4-Dihydro-2H-pyran-3-yl, 5,6-Dihydro-2H-pyran-3-yl, 2,3-Dihydro-4H-pyran-3-yl, 5,6-Dihydro-4H-pyran-3-yl, 3,4-Dihydro-2H-pyran-4-yl, 5,6-Dihydro-2H-pyran-4-yl, 2,3-Dihydro-4H-pyran-4-yl, 3,4-Dihydro-2H-thiopyran-2-yl, 5,6-Dihydro-2H-thiopyran-2-yl, 2,3-Dihydro-4H-thiopyran-2-yl, 5,6-Dihydro-4H-thiopyran-2-yl, 3,4-Dihydro-2H-thiopyran-3-yl, 5,6-Dihydro-2H-thiopyran-3-yl, 2,3-Dihydro-4H-thiopyran-3-yl, 5,6-Dihydro-4H-thiopyran-3-yl, 3,4-Dihydro-2H-thiopyran-4-yl, 5,6-Dihydro-2H-thiopyran-4-yl und 2,3-Dihydro-4H-thiopyran-4-yl, wobei dieser Ring ein bis drei Substituenten tragen kann, ausgewählt aus einer Gruppe bestehend aus: Hydroxy,
   Halogen wie vorstehend genannt, insbesondere Fluor oder Chlor C₁-C₄-Alkyl wie vorstehend genannt, insbesondere Methyl, C₁-C₄-Halogenalkyl wie vorstehend genannt, insbesondere Trifluormethyl,
   C₁-C₄-Alkoxy wie vorstehend genannt, insbesondere Methoxy und C₁-C₄-Alkylthio wie vorstehend genannt, insbesondere Methylthio;
   besonders bevorzugt ist ein 6-gliedriger gesättigter Heterocyclus, welcher neben Kohlenstoffringgliedern ein oder zwei Sauerstoff- und/oder Schwefelatome enthalten kann;
- ein 5-gliedriger Heteroaromat, welcher neben Kohlenstoffringgliedern ein oder zwei Stickstoffatome und ein Sauerstoff- oder ein Schwefelatom enthält wie 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4- Triazol-3-yl, 1,3,4-Oxadiazol-2-yl, 1,3,4-Thiadiazol-2-yl und 1,3,4-Triazol-2-yl, insbesondere 5-Isoxazolyl wobei dieser Ring ein bis drei Substituenten tragen kann, ausgewählt aus einer Gruppe bestehend aus: Cyano,
   Halogen wie vorstehend genannt, insbesondere Fluor oder Chlor,
   C₁-C₄-Alkyl wie vorstehend genannt, vorzugsweise 1-Methylethyl, Methyl, insbesondere 1-Methylethyl,
   C₁-C₄-Halogenalkyl wie vorstehend genannt, insbesondere Trifluormethyl,
   C₁-C₄-Alkoxy wie vorstehend genannt, vorzugsweise 1-Methylethoxy, Methoxy, insbesondere Methoxy,
   C₁-C₄-Alkylthio wie vorstehend genannt, insbesondere Methylthio,
   C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, i-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl. 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2.3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise Ethenyl, 2-Propenyl, 2-Butenyl, insbesondere 2-Propenyl, C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 2-Propinyl, 2-Butinyl, insbesondere 2-Propinyl und
   C₁-C₄-Alkoxy-C₁-C₄-alkyl wie durch Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy substituiertes C₁-C₄-Alkyl wie vorstehend genannt, insbesondere Ethyl;
   unter den Substituenten am 5-gliedrigen Heteroaromat ist C₁-C₄-Alkyl besonders bevorzugt;
- Phenyl oder Pyridyl, wobei diese Ringe ein bis drei Substituenten tragen können, ausgewählt aus einer Gruppe bestehend aus:
   Nitro,
   Cyano,
   Halogen wie vorstehend genannt, insbesondere Fluor oder Chlor,
   C₁-C₄-Alkyl wie vorstehend genannt, insbesondere Methyl, C₁-C₄-Halogenalkyl wie vorstehend genannt, insbesondere Trifluormethyl,
   C₁-C₄-Alkoxy wie vorstehend genannt, insbesondere Methoxy,
   C₁-C₄-Alkylthio wie vorstehend genannt, insbesondere Methylthio,
   C₃-C₆-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy,4-Methyl-4-pentenyloxy, 1,1-Dimethyl-2-butenyloxy, 1,1-Di-methyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 3,3-Dimethyl-2-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, insbesondere 2-Propenyloxy,
   C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, insbesondere 2-Propinyloxy,
   und einem Substituenten NR^{a}R^{b}, wobei
      R^{a} für Wasserstoff,
      für C₁-C₄-Alkyl wie vorstehend genannt, insbesondere Methyl,
      für C₃-C₆-Alkenyl wie vorstehend genannt, insbesondere 2-Propenyl, 2-Butenyl oder
      für C₃-C₆-Alkinyl wie vorstehend genannt, insbesondere 2-Propinyl, 2-Butinyl steht und
      R^{b} für Wasserstoff
      für C₁-C₄-Alkyl wie vorstehend genannt, insbesondere Methyl,
      für C₃-C₆-Alkenyl wie vorstehend genannt, insbesondere 2-Propenyl, 2-Butenyl,
      für C₃-C₆-Alkinyl wie vorstehend genannt, insbesondere 2-Propinyl, 2-Butinyl,
      für C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, insbesondere Methylcarbonyl, Ethylcarbonyl oder
      für Benzoyl steht, wobei der Phenylring seinerseits ein bis drei der folgenden Reste tragen kann:
      - Nitro,
      - Cyano,
      - Halogen wie vorstehend genannt, insbesondere Fluor oder Chlor,
      - C₁-C₄-Alkyl wie vorstehend genannt, insbesondere Methyl
      - C₁-C₄-Halogenalkyl wie vorstehend genannt, insbesondere Trilfuormethyl,
      - C₁-C₄-Alkoxy wie vorstehend genannt, insbesondere Methoxy und/oder
      - C₁-C₄-Alkylthio wie vorstehend genannt, insbesondere Methylthio;
      besonders bevorzugt ist Phenyl, das ein bis drei Nitro-, Halogen- und/oder C₁-C₄-Alkylsubstituenten tragen kann;
      R⁴ Wasserstoff;
      C₁-C₁₀-Alkyl, besonders C₁-C₆-Alkyl wie vorstehend genannt, insbesondere Methyl,
      C₁-C₁₀-Halogenalkyl wie vorstehend im allgemeinen und im besonderen genannt, insbesondere Trilfuormethyl,
      C₃-C₁₀-Alkenyl, besonders C₃-C₆-Alkenyl wie vorstehend genannt, C₃-C₁₀-Halogenalkenyl wie vorstehend im allgemeinen und im besonderen genannt, insbesondere 2-Propenyl,
      C₃-C₁₀-Alkinyl wie vorstehend genannt, Insbesondere 2-Propinyl, C₃-C₁₀-Halogenalkinyl wie vorstehend im allgemeinen und im besonderen genannt,
      C₁-C₁₀-Alkylcarbonyl, besonders C₁-C₆-Alkylcarbonyl wie vorstehend genannt, insbesondere Methylcarbonyl,
      C₁-C₁₀-Halogenalkylcarbonyl wie vorstehend im allgemeinen und im besonderen genannt;
      Benzoyl, wobei der Phenylring ein bis drei der folgenden Gruppen tragen kann:
      - Nitro,
      - Cyano,
      - Halogen wie vorstehend genannt, insbesondere Fluor und Chlor,
      - C₁-C₄-Alkyl wie vorstehend genannt, insbesondere Methyl,
      - C₁-C₄-Halogenalkyl wie vorstehend genannt, insbesondere Trifluormethyl,
      - C₁-C₄-Alkoxy wie vorstehend genannt, insbesondere Methoxy und/oder
      - C₁-C₄-Alkylthio wie vorstehend genannt, insbesondere Methylthio;
      -S(=O)₂-R^{c}; -P(=O)(OR^{d})(OR^{e}) oder -SiR^{f}R^{g}R^{h}, wobei
      R^{c}, R^{d}, R^{e}, R^{f}, R^{g} und R^{h} unabhängig voneinander
      für C₁-C₁₀-Alkyl, besonders C₁-C₆-Alkyl wie vorstehend genannt, vorzugsweise Methyl, Ethyl, 1,1-Dimethylethyl, insbesondere Methyl,
      für C₁-C₁₀-Halogenalkyl wie vorstehend im allgemeinen und im besonderen genannt, oder
      für Phenyl stehen, wobei der Phenylring ein bis drei der folgenden Reste tragen kann:
         - Nitro,
         - Cyano,
         - Halogen wie vorstehend genannt, insbesondere, Fluor oder Chlor,
         - C₁-C₄-Alkyl wie vorstehend genannt, insbesondere Methyl
         - C₁-C₄-Halogenalkyl wie vorstehend genannt, insbesondere Trifluormethyl
         - C₁-C₄-Alkoxy wie vorstehend genannt, insbesondere Methoxy und/oder
         - C₁-C₄-Alkylthio wie vorstehend genannt, insbesondere Methylthio;
         besonders bevorzugt bedeutet R⁴ Wasserstoff.

Nicht von der Erfindung umfaßt werden solche Cyclohexenonoximether der Formel I, in denen R³ für 2-(Ethylthio)propyl steht, wenn R¹ Ethyl, R² 3-Chlor-2-propenyl und R⁴ Wasserstoff bedeuten.

Teil der Erfindung sind jedoch auch die landwirtschaftlich brauchbaren Salze von I sowie die Ester aus C₁-C₁₀-Carbonsäuren oder anorganischen Säuren und den Verbindungen I. Üblicherweise kommt es hierbei auf die Art des Salzes oder des Esters nicht an. Allgemein kommen die Salze von solchen Basen und solchen Estern in Betracht, welche die herbizide Wirkung der Verbindungen I nicht negativ beeinträchtigen.

Landwirtschaftlich brauchbare Salze der Verbindungen I sind beispielweise Alkalimetallsalze, insbesondere Natrium- und Kaliumsalze, Erdalkalimetallsalze wie insbesondere Calzium-, Magnesium- und Bariumsalze, Mangan-, Kupfer-, Zink- oder Eisensalze sowie Ammoniumsalze wie Tetraalkyl- und Benzyltrialkylammoniumsalze, Phosphonium-, Sulfoniumsalze wie Trialkylsulfoniumsalze oder Sulfoxoniumsalze.

Landwirtschaftlich brauchbare Ester sind z.B. die Ester der Cyclohexenonoximether I mit
- C₁-C₁₀-Fettsäuren, besonders C₁-C₆-Alkylcarbonsäuren wie Methylcarbonsäure (Essigsäure), Ethylcarbonsäure (Propionsäure), Propylcarbonsäure (Buttersäure), 1-Methylethylcarbonsäure (Isobuttersäure), Butylcarbonsäure, 1-Methylpropylcarbonsäure, 2-Methylpropylcarbonsäure, 1,1-Dimethylethylcarbonsäure, Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2,2-Dimethylpropylcarbonsäure, 1-Ethylpropylcarbonsäure, Benzoesäure und entsprechende am Phenylkern substituierte Derivate, Hexylcarbonsäure, 1-Methylpentylcarbonsäure, 2-Methylpentylcarbonsäure, 3-Methylpentylcarbonsäure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethylbutylcarbonsäure, 1-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methylpropylcarbonsäure und 1-Ethyl-2-methylpropylcarbonsäure;
- C₁-C₂₀-Sulfonsäuren, besonders C₁-C₆-Alkylsulfonsäuren wie Methylsulfonsäure, Ethylsulfonsäure, Propylsulfonsäure, 1-Methylethylsulfonsäure, Butylsulfonsäure, 1-Methylpropylsulfonsäure, 2-Methylpropylsulfonsäure, 1,1-Dimethylethylsulfonsäure, Pentylsulfonsäure, 1-Methylbutylsulfonsäure, 2-Methylbutylsulfonsäure, 3-Methylbutylsulfonsäure, 1,1-Dimethylpropylsulfonsäure, 1,2-Dimethylpropylsulfonsäure, 2,2-Dimethylpropylsulfonsäure, 1-Ethylpropylsulfonsäure, Benzolsulfonsäure und entsprechende am Phenylkern substituierte Derivate, Hexylsulfonsäure, 1-Methylpentylsulfonsäure, 2-Methylpentylsulfonsäure, 3-Methylpentylsulfonsäure, 4-Methylpentylsulfonsäure, 1,1-Dimethylbutylsulfonsäure, 1,2-Dimethylbutylsulfonsäure, 1,3-Dimethylbutylsulfonsäure, 2,2-Dimethylbutylsulfonsäure, 2,3-Dimethylbutylsulfonsäure, 3,3-Dimethylbutylsulfonsäure, 1-Ethylbutylsulfonsäure, 2-Ethylbutylsulfonsäure, 1,1,2-Trimethylpropylsulfonsäure, 1,2,2-Trimethylpropylsulfonsäure, 1- Ethyl-1-methylpropylsulfonsäure und 1-Ethyl-2-methylpropylsulfonsäure oder
- C₁-C₂₀-Phosphonsäuren, besonders C₁-C₆-Alkylphosphonsäuren wie Methylphosphonsäure, Ethylphosphonsäure, Propylphosphonsäure, 1-Methylethylphosphonsäure, Butylphosphonsäure, 1-Methylpropylphosphonsäure, 2-Methylpropylphosphonsäure, 1,1-Dimethylethylphosphonsäure, Pentylphosphonsäure, 1-Methylbutylphosphonsäure, 2-Methylbutylphosphonsäure, 3-Methylbutylphosphonsäure, 1,1-Dimethylpropylphosphonsäure, 1,2-Dimethylpropylphosphonsäure, 2,2-Dimethylpropylphosphonsäure, 1-Ethylpropylphosphonsäure, Benzolphosphonsäure und entsprechende am Phenylkern substituierte Derivate, Hexylphosphonsäure, 1-Methylpentylphosphonsäure, 2-Methylpentylphosphonsäure, 3-Methylpentylphosphonsäure, 4-Methylpentylphosphonsäure, 1,1-Dimethylbutylphosphonsäure, 1,2-Dimethylbutylphosphonsäure, 1,3-Dimethylbutylphosphonsäure, 2,2-Dimethylbutylphosphonsäure, 2,3-Dimethylbutylphosphonsäure, 3,3-Dimethylbutylphosphonsäure, 1-Ethylbutylphosphonsäure, 2-Ethylbutylphosphonsäure, 1,1,2-Trimethylpropylphosphonsäure, 1,2,2-Trimethylpropylphosphonsäure, 1-Ethyl-1-methylpropyl-phosphonsäure und 1-Ethyl-2-methylpropylphosphonsäure.

Die folgenden Cyclohexenonoximether Ia.01 bis Id.48 der Tabellen 1 und 2 sind besonders bevorzugt:

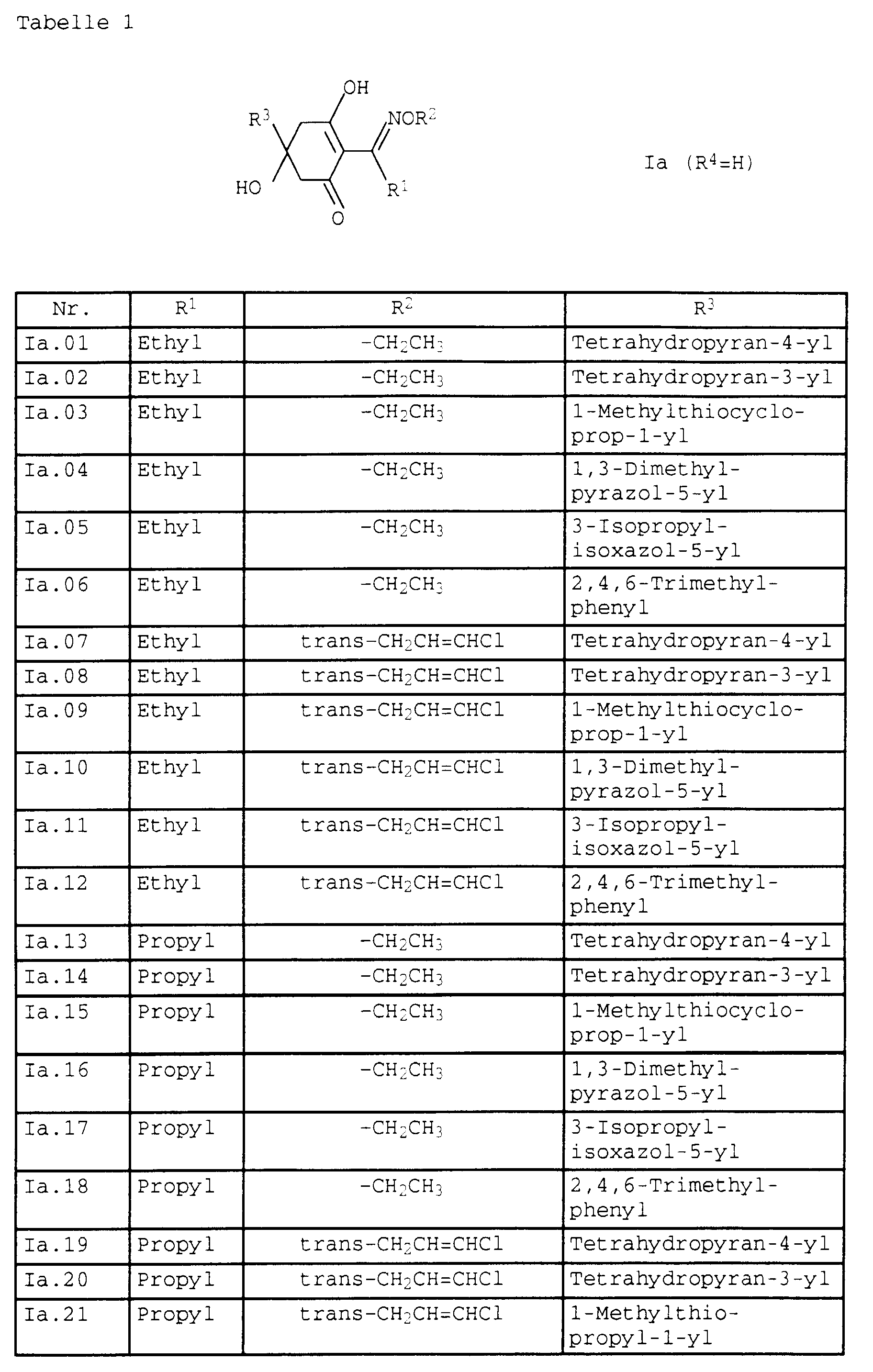

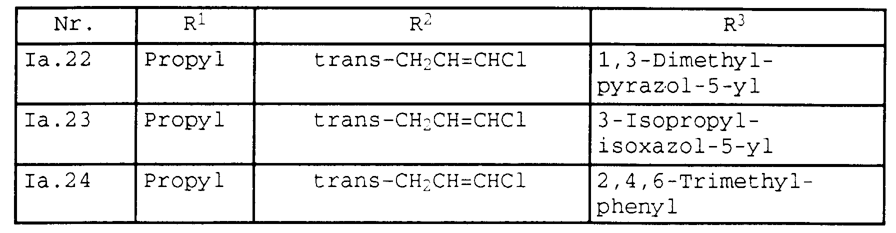

Die Cyclohexenonoximether Ib.01 bis Ib.24 sind bis auf R⁴, das in diesem Fall Methyl bedeutet, mit den entsprechenden Verbindungen Ia.01 bis Ia.24 identisch; die Cyclohexenonoximether Ic.01 bis Ic.24 sind bis auf R⁴, das in diesem Fall Methylcarbonyl bedeutet, mit den entsprechenden Verbindungen Ia.01 bis Ia.24 identisch.

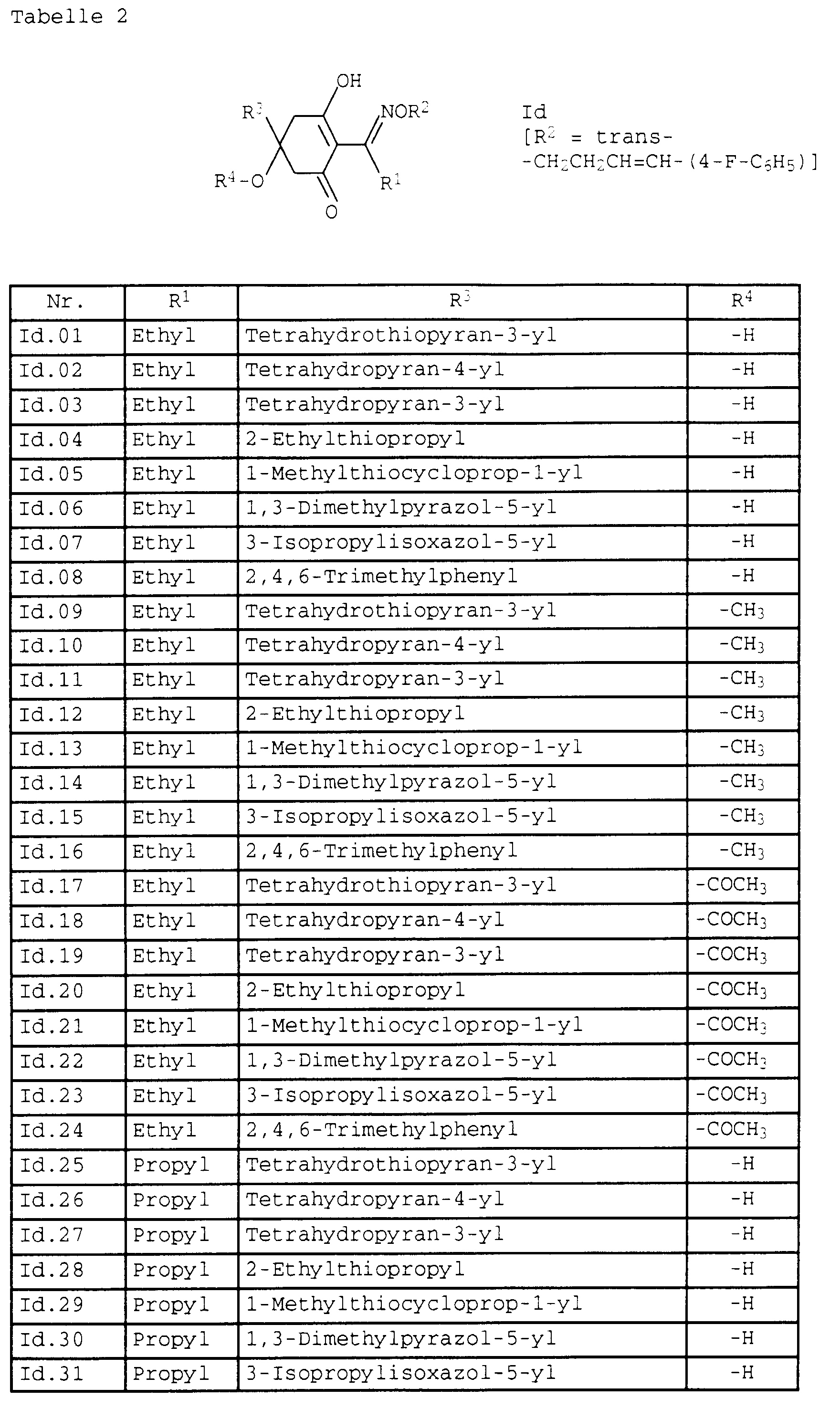

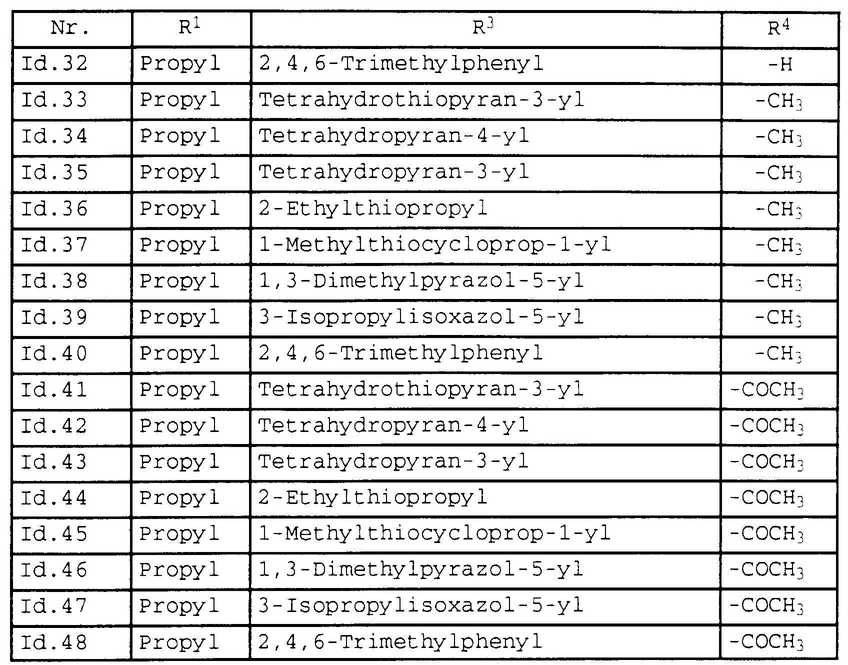

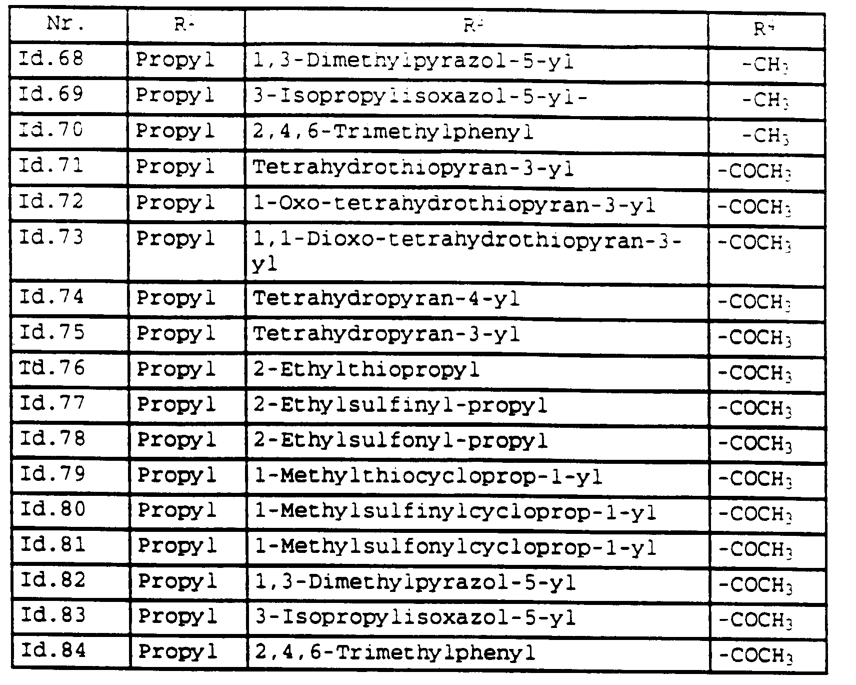

Die Cyclohexenonoximether Ie.01 bis Ie.48 sind bis auf R², das in diesem Fall -CH₂CH₂CH₂CH₂-(4-F-C₆H₄) bedeutet, mit den entsprechenden Verbindungen Id.01 bis Id.48 identisch;
die Cyclohexenonoximether If.01 bis If.48 sind bis auf R², das in diesem Fall -CH₂CH(CH₃)-O-(4-Cl-C₆H₄) bedeutet, mit den entsprechenden Verbindungen Id.01 bis Id.48 identisch;
die Cyclohexenonoximether Ig.01 bis Ig.48 sind bis auf R², das in diesem Fall -CH₂CH₂-O-(2,4-F₂-C₆H₃) bedeutet, mit den entsprechenden Verbindungen Id.01 bis Id.48 identisch;
die Cyclohexenonoximether Ih.01 bis Ih.48 sind bis auf R², das in diesem Fall -CH₂-(5-Cl-thien-2-yl) bedeutet, mit den entsprechenden Verbindungen Id.01 bis Id.48 identisch;
die Cyclohexenonoximether Ii.01 bis Ii.48 sind bis auf R², das in diesem Fall -CH₂CH₂CH₂CH₂-(thien-2-yl) bedeutet, mit den entsprechenden Verbindungen Id.01 bis Id.48 identisch.

Die Cyclohexenonoximether I sowie deren Salze und Ester eignen sich - sowohl als Isomerengemische als auch in Form der reinen Isomeren - als Herbizide, Insbesondere zur Bekämpfung von Pflanzenarten aus der Familie der Gräser (Gramineen).

Im allgemeinen sind sie verträglich und somit selektiv in breitblättrigen Kulturen sowie in monokotylen (einkeimblättrigen) Gewachsen, welche nicht zu den Gramineen zählen. Einige der erfindungsgemäßen cyclohexenonoximether I sind auch zur selektiven Bekampfung von unerwünschten Gräsern in Gramineenkulturen geeignet.

Die Cyclohexenonoximether I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Losungsmittel, wie N-Methylpyrrolidon oder Wasser in Betracht.

Waßrige Anwendungsformen konnen aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsaure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel konnen durch Mischen oder gemeinsames Vermahlen der wirksamen Substanzen mit einem festen Tragerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemaßen Verbindungen I konnen beispielsweise wie folgt formuliert werden:
- I: 20 Gew.-Teile der Verbindung Nr. 1.26 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Olsäure-N-monoethanolamid, 5 Gew.-Teilen des Anlagerungsproduktes von 40 Mol Ethylenoxid an 1 Mol Ricinusöl besteht. Durch Ausgießen und feines Verteilen der Losung in 100 000 Gew.-Teilen Wasser erhält man eine wäßrige Dispersion, die 0,02 Gew.-% des Wirkstoffs enthält.
- II: 20 Gew.-Teile der Verbindung Nr. 1.04 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
- III: 20 Gew.-Teile des Wirkstoffs Nr. 1.06 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine waßrige Dispersion, die 0,02 Gew.-% des Wirkstoffs enthält.
- IV: 20 Gew.-Teile des Wirkstoffs Nr. 1.25 werden mit 3 Gew.-Teilen des Natriumsalzes der Diisobutylnaphthalin-α-sulfonsäure 17 Gew.-Teilen des Natriumsalzes einer Ligninsulfonsaure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
- V: 3 Gew.-Teile des Wirkstoffs Nr. 1.24 werden mit 97 Gew.-Teilen feinteiligem Kaolin vermischt. Man erhält auf diese Weise ein Stäubemittel, das 3 Gew.-% des Wirkstoffs enthält.
- VI: 20 Gew.-Teile des Wirkstoffs Nr. 1.18 werden mit 2 Gew.-Teilen Calciumsalz der Dodecylbenzolsulfonsaure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgerate so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die Cyclohexenonoximether I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis Guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicaco sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Zur Verbreiterung des Wirkungsspektrums und zur Erzielung synergistischer Effekte konnen die Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäure sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Olkonzentrate zugesetzt werden.

### Synthesebeispiele

Die in den nachstehend gegebenen Beispielen wiedergegebenen Synthesevorschriften wurden unter Abwandlung der Edukte noch zur Herstellung weiterer Cyclohexenonoximether der Formel I verwendet. Die so erhaltenen Verbindungen sind in den anschließenden Tabellen mit physikalischen Daten aufgeführt.

### Exemplarische Herstellvorschrift

### 3,5-Dihydroxy-2-propionyl-5-(2H-tetrahydropyran-4-yl)-2-cyclohexen-1-on (Nr. IV.1)

Unter Stickstoff tropfte man zu einer Lösung aus 0,9 mol Lithiumdiisopropylamid (hergestellt aus 90,9 g Diisopropylamin und 384 g einer 15 %igen Losung aus n-Butyllithium in Hexan) in 800 ml THF bei (-70)°C 46,8 g (0,3 mol) 3-Acetyl-2,4-hexandion. Man ruhrte 1,5 Std. bei (-70)°C nach, ließ die Reaktionsmischung auf Raumtemperatur aufwarmen und tropfte anschließend bei (-70)°C 44,6 g (0,3 mol) 2H-Tetranyropyran-4-carbonsaurechlorid hinzu. Man ruhrte 1,5 h bei (-70)°C nach und ließ über Nacht auf ca. 20°C aufwarmen. Die Reaktionsmischung wurde danach in 800 ml Eiswasser eingerührt und mit konzentrierter Salzsaure unterhalb 15°C auf pH 5 (pH-Meßgerat) eingestellt. Die org. Phase wurde abgetrennt, die waßrige Phase noch zweimal mit MTB extrahiert. Die vereinigten organischen Phasen wurden über Na₂SO₄ getrocknet und unter reduziertem Druck eingeengt. Das erhaltene dunkle Öl wurde in 300 ml Diisopropylether ausgerührt und der ausgefallene Niederschlag abgetrennt, mit wenig Diisopropylether gewaschen und getrocknet. Ausbeute: 7,9 g (10 %) hellgelber Feststoff; Schmp. 148 bis 153°C.
¹H-NMR (200 MHz, in CDCl₃) : δ [ppm] = 1.15 (t,3H), 1.30-1.80 (m,5H), 2.50-2.80 (m,4H), 3.10 (q,2H), 3.35 (m,2H), 4,05 (m,2H), 4.50 (bs,1H), 18.20 (bs,1H).

### 2-[1-[[(E)-3-Chlor-2-propen-1-yl-oxy]imino]propyl]-3,5-dihydroxy-5-(2H-tetrahydropyran-4-yl)-2-cyclohexen-1-on (I.24)

6,0 g (0,022 mol) Cyclohexenon (Nr. IV.1) 3,6 g (0,025 mol) O-[(E)-3-Chlor-2-propen-1-yl]hydroxylamin Hydrochlorid und 2,1 g (0,025 mol) NaHCO₃ wurden 24 Std. in 100 ml Methanol bei ca. 20 bis 25°C gerührt. Man engte unter reduziertem Druck ein, nahm den Rückstand in Wasser auf, säuerte mit 10 %iger Salzsäure an und extrahierte mit Methylenchlorid. Die organische Phase wurde abgetrennt, über Natriumsulfat getrocknet und unter reduziertem Druck eingeengt. Ausbeute: 7,9 g (100 %) hellbrauner Feststoff; Schmp. 129-132°C.
¹H-NMR (200 MHz, in CDCl₃) : δ [ppm] = 1.15 (t,3H), 1.30-1.80 (m,5H), 2.00 (bs,1H), 2.50-2.80 (m,4H), 2.90 (q,2H), 3.40 (m,2H), 4.05 (m,2H), 4.50 (d,2H), 6.10 (m,1H), 6.35 (d,1H), 14.50 (bs,1H).
- [Abkurzungen:: THF = Tetrahydrofuran;
MTB = Methyl-tert.-butylether]

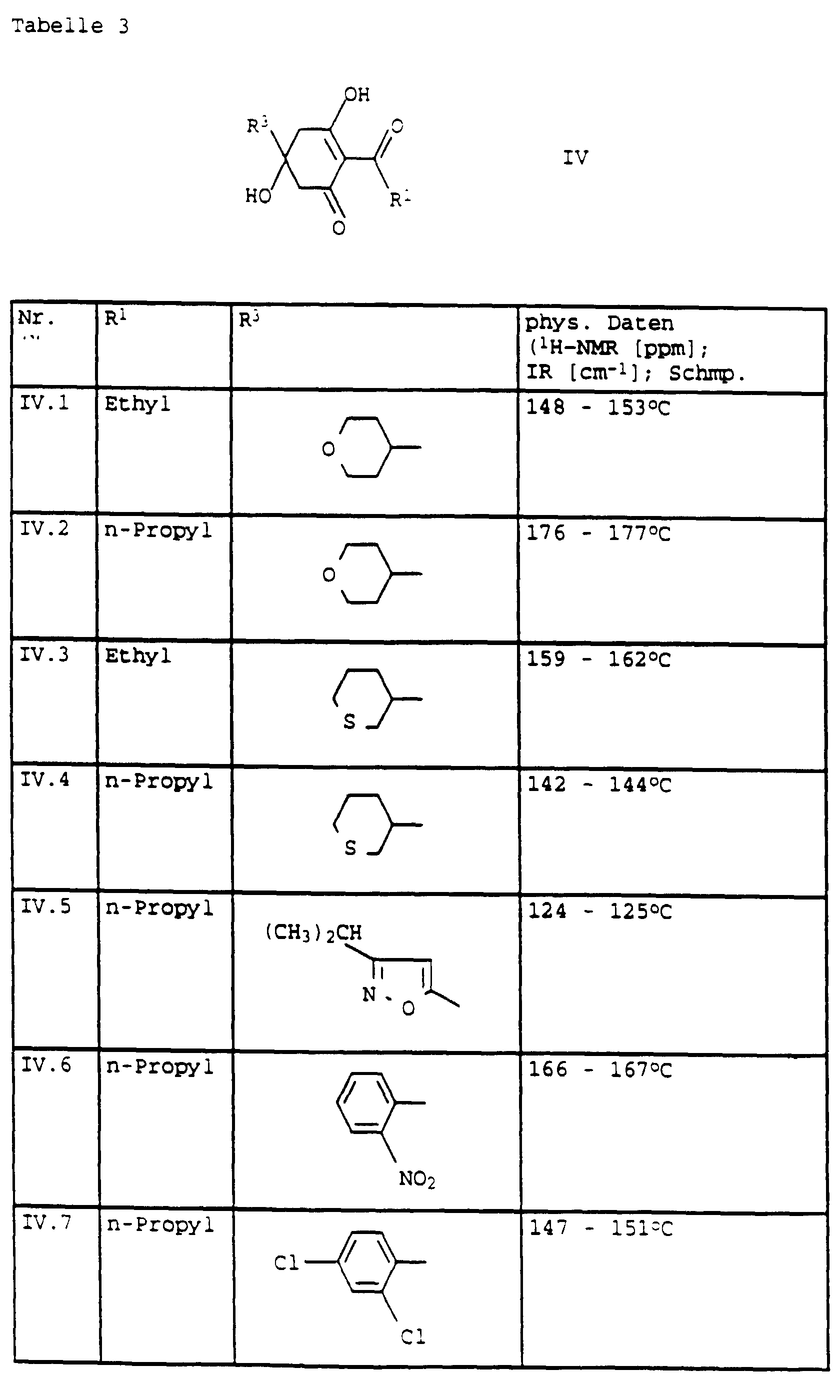

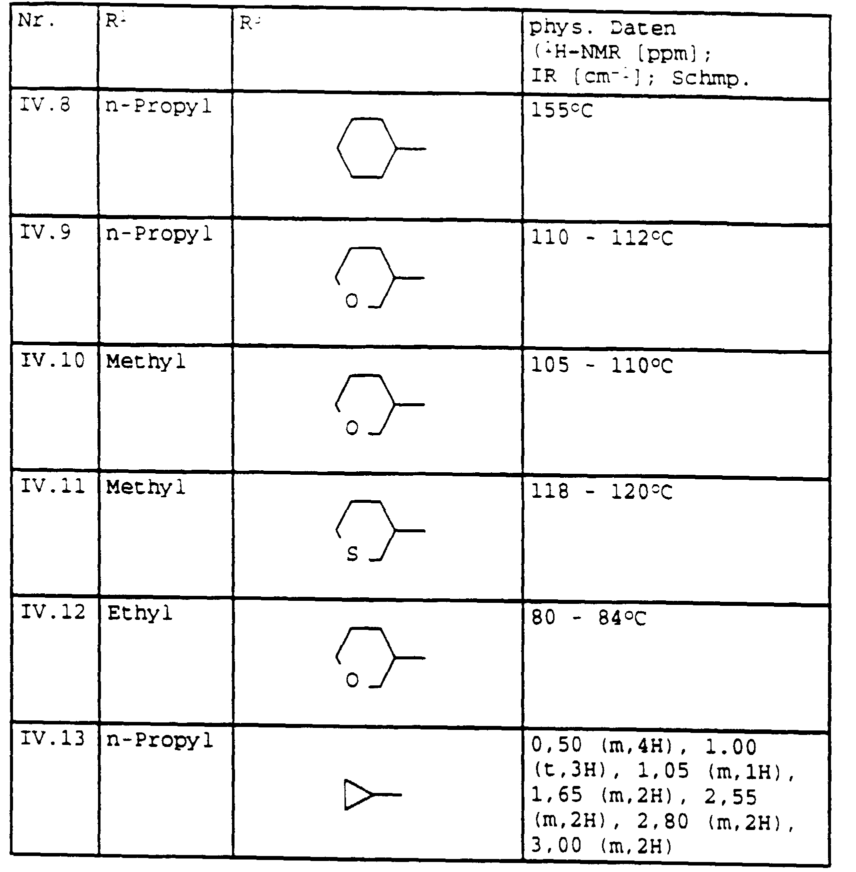

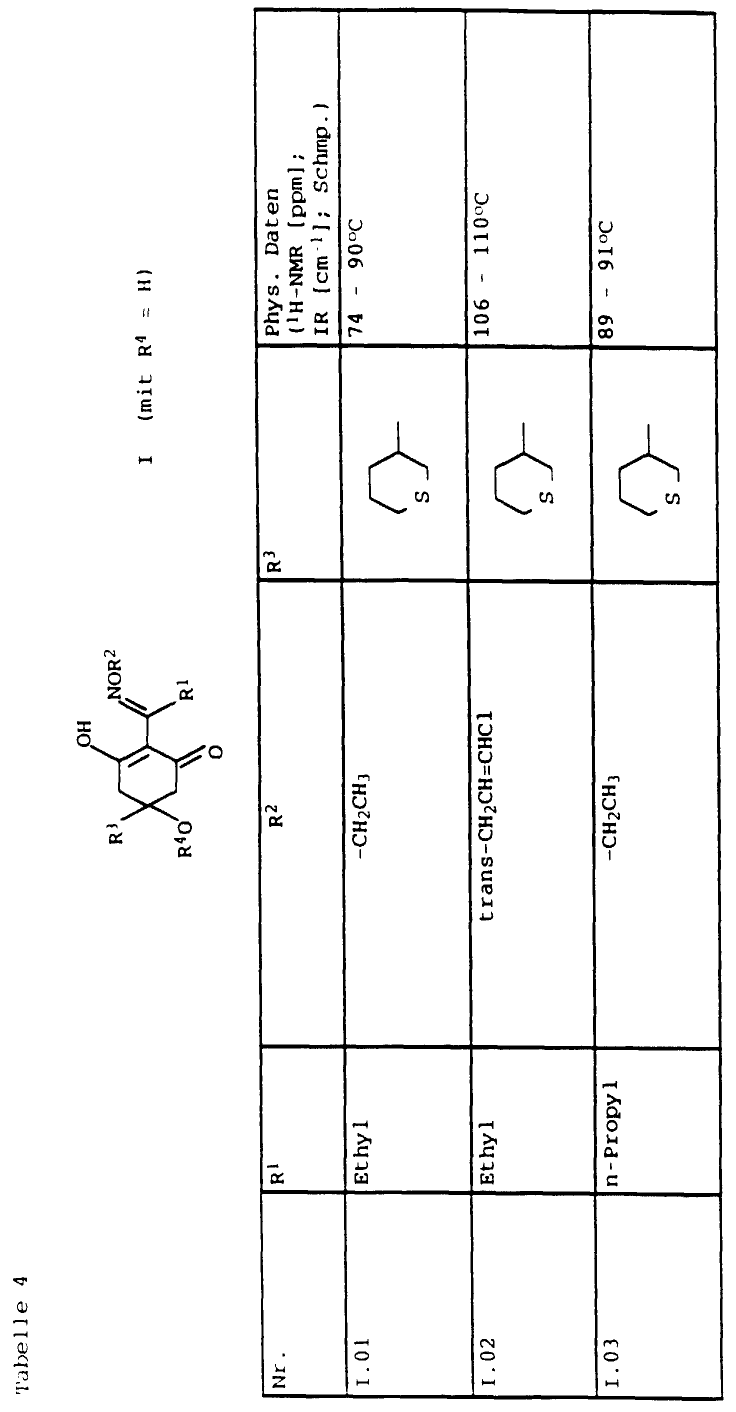

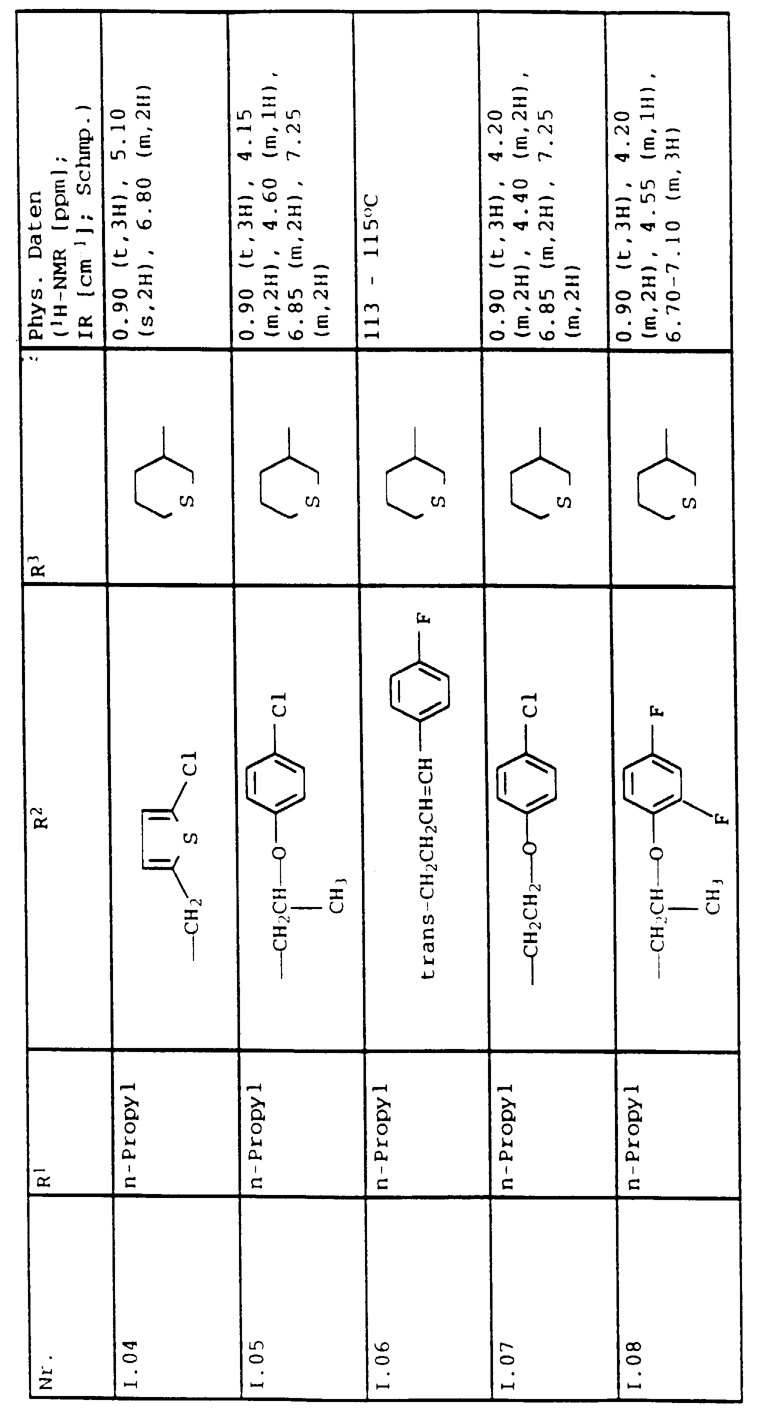

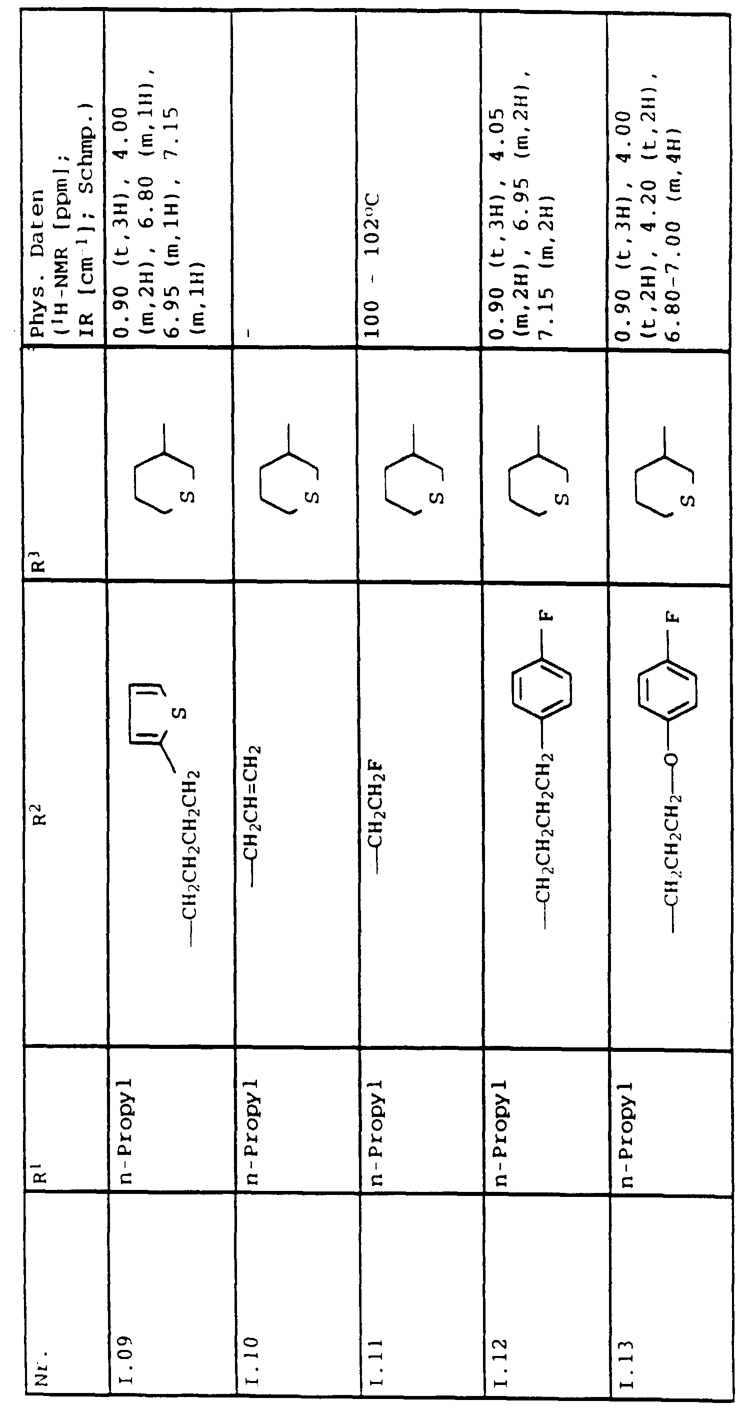

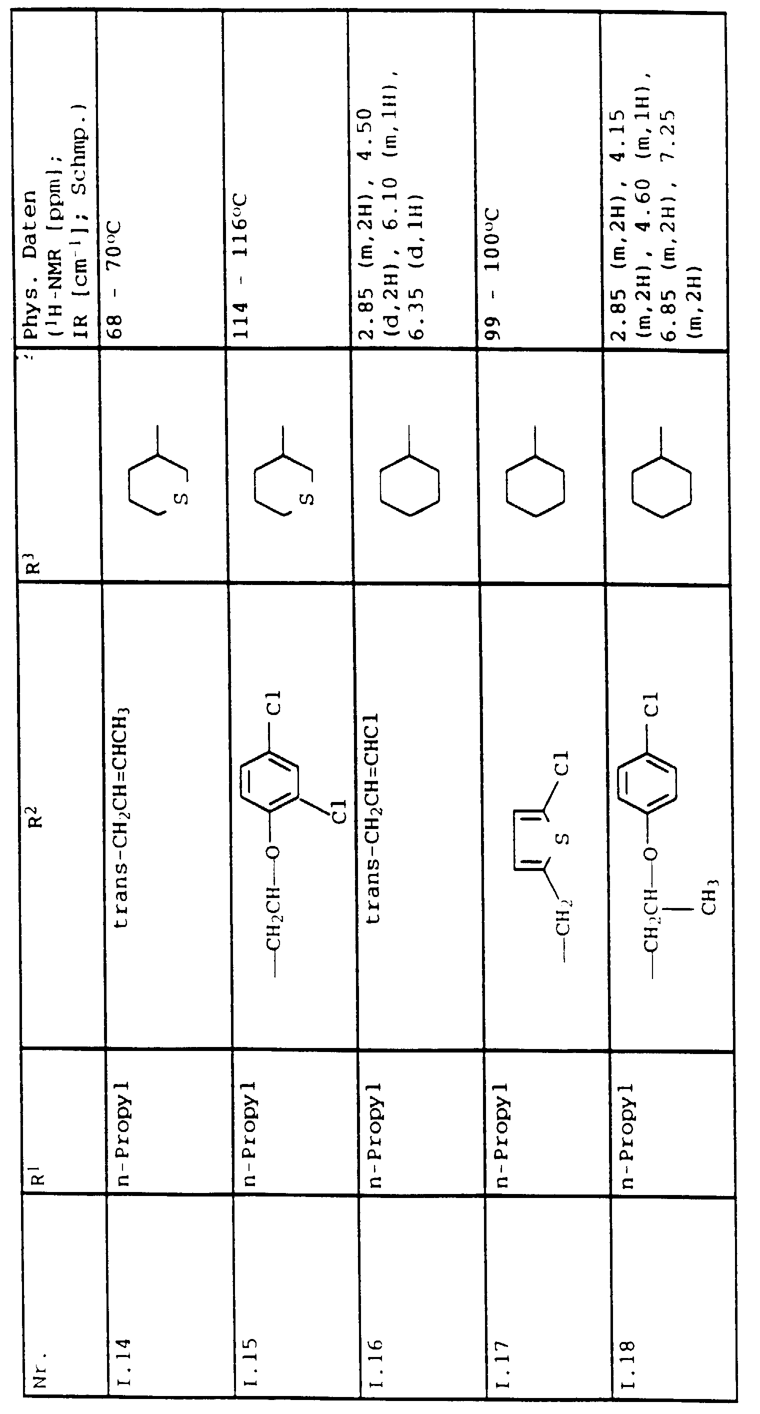

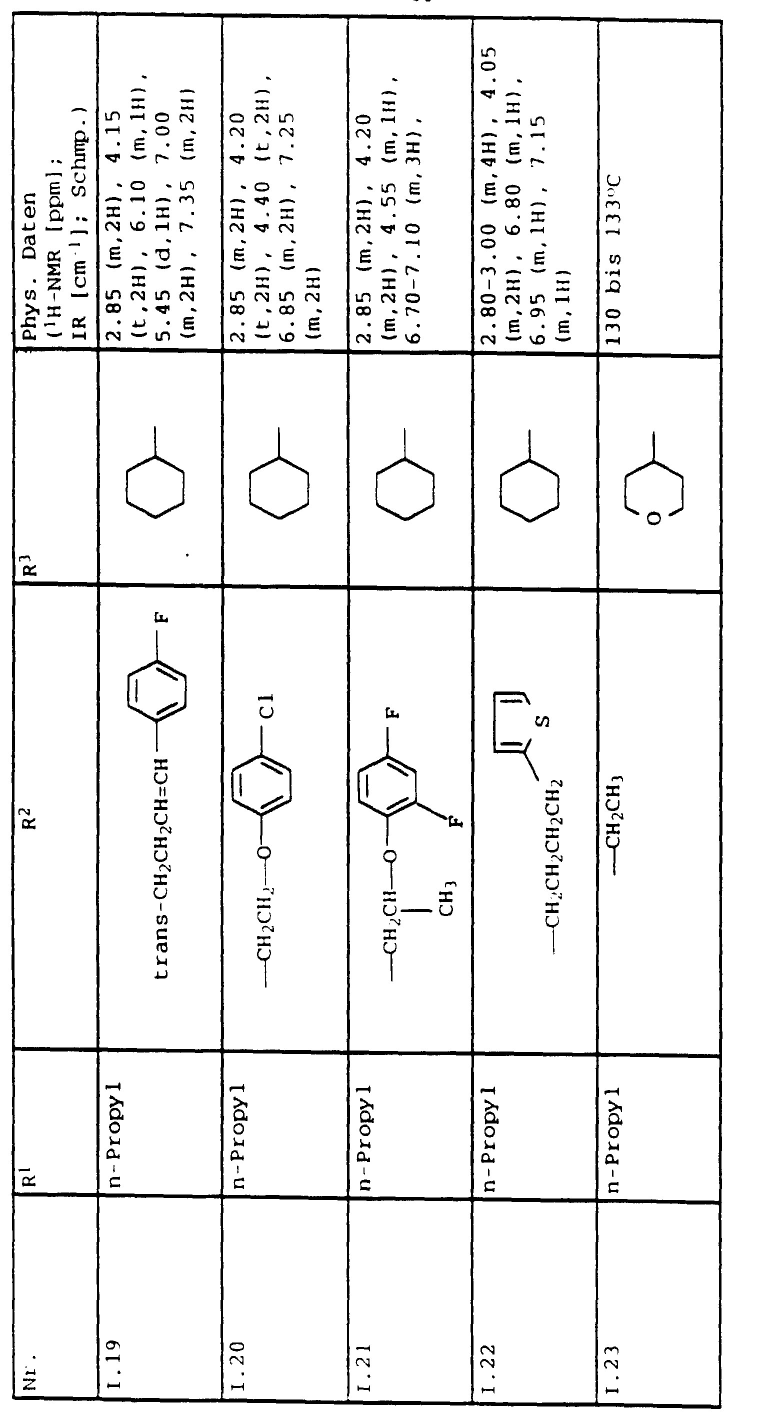

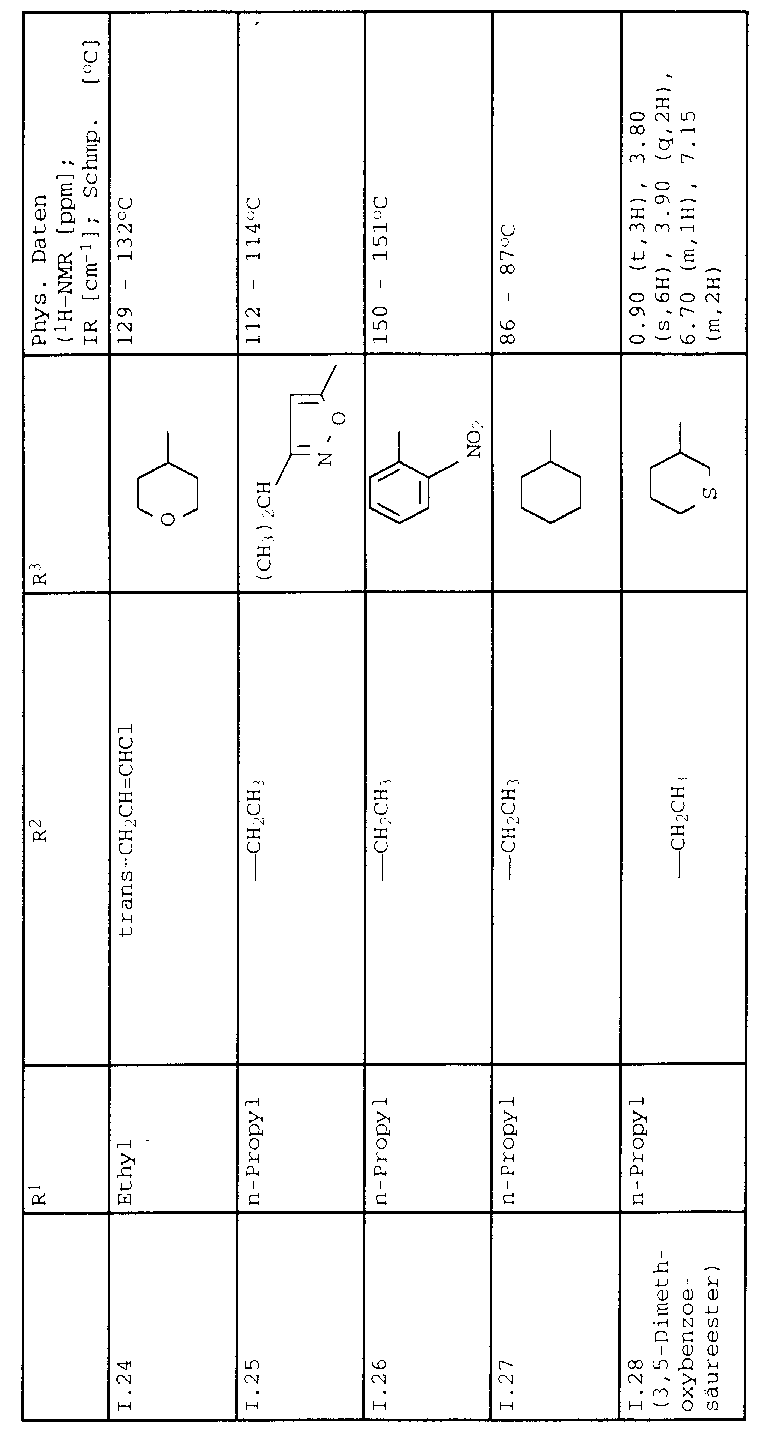

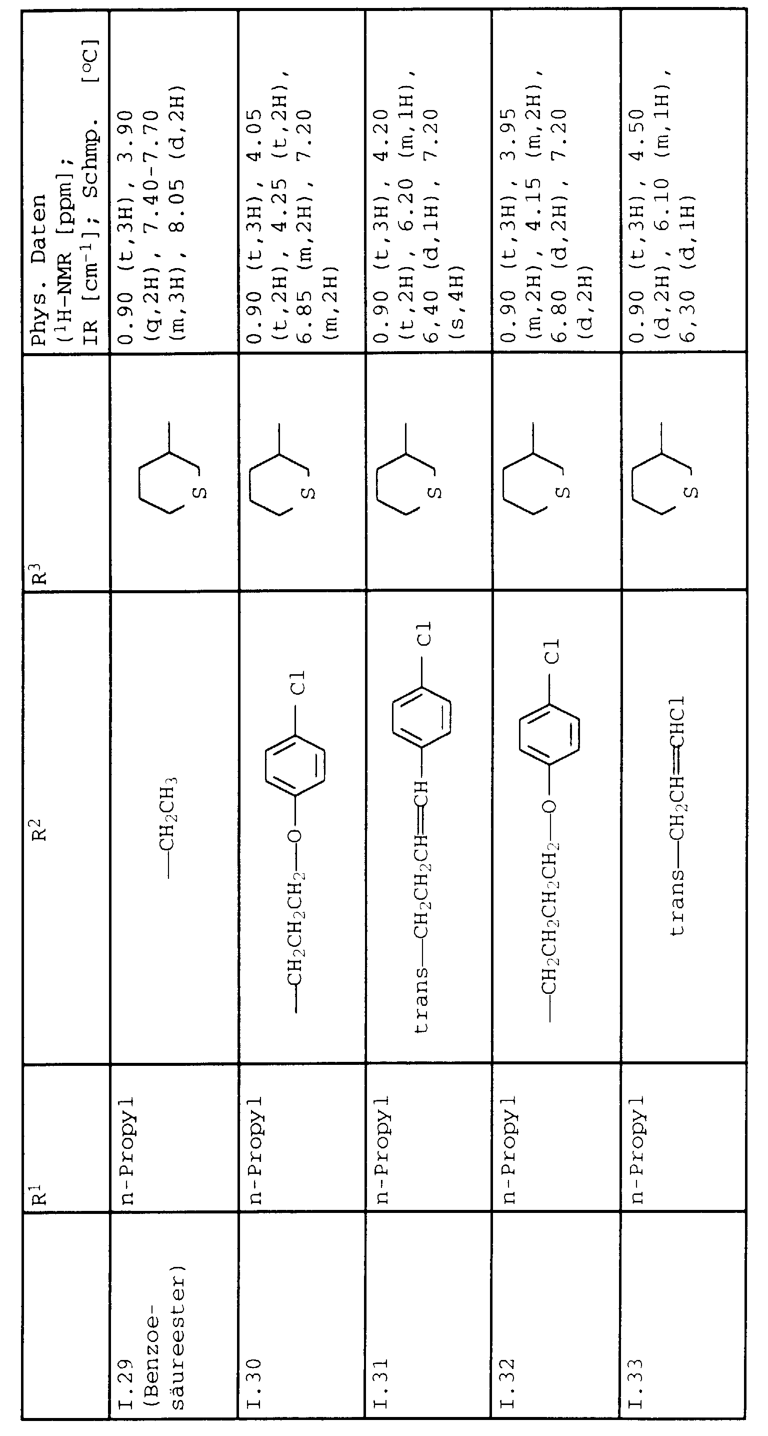

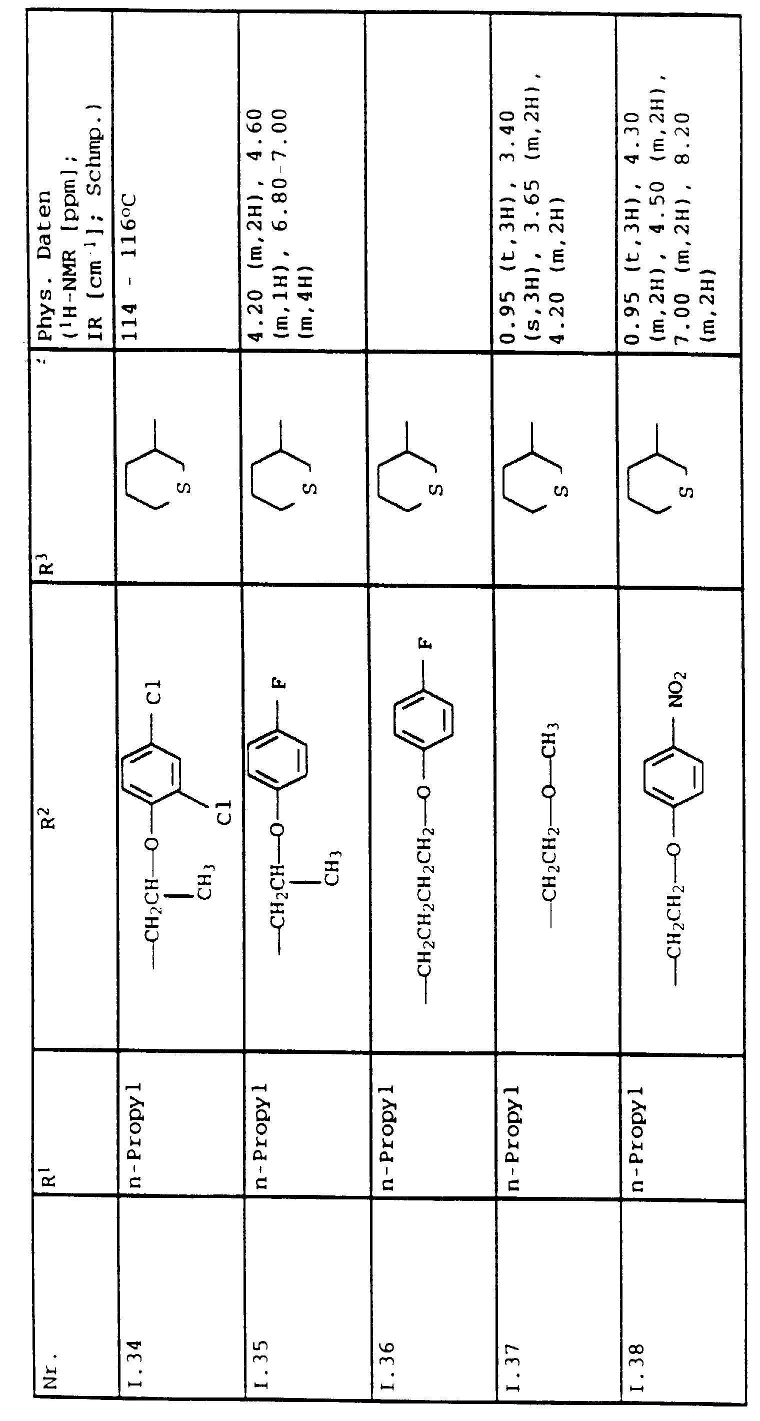

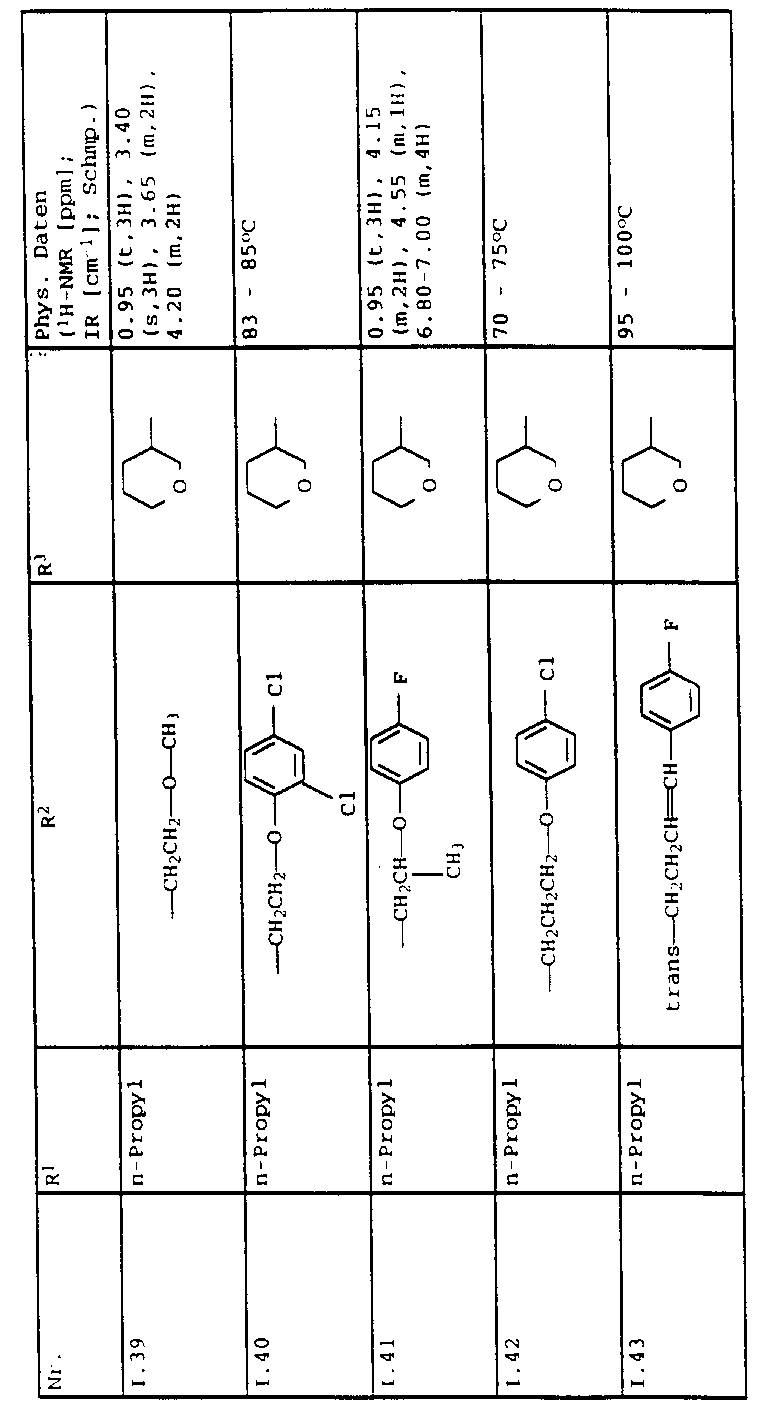

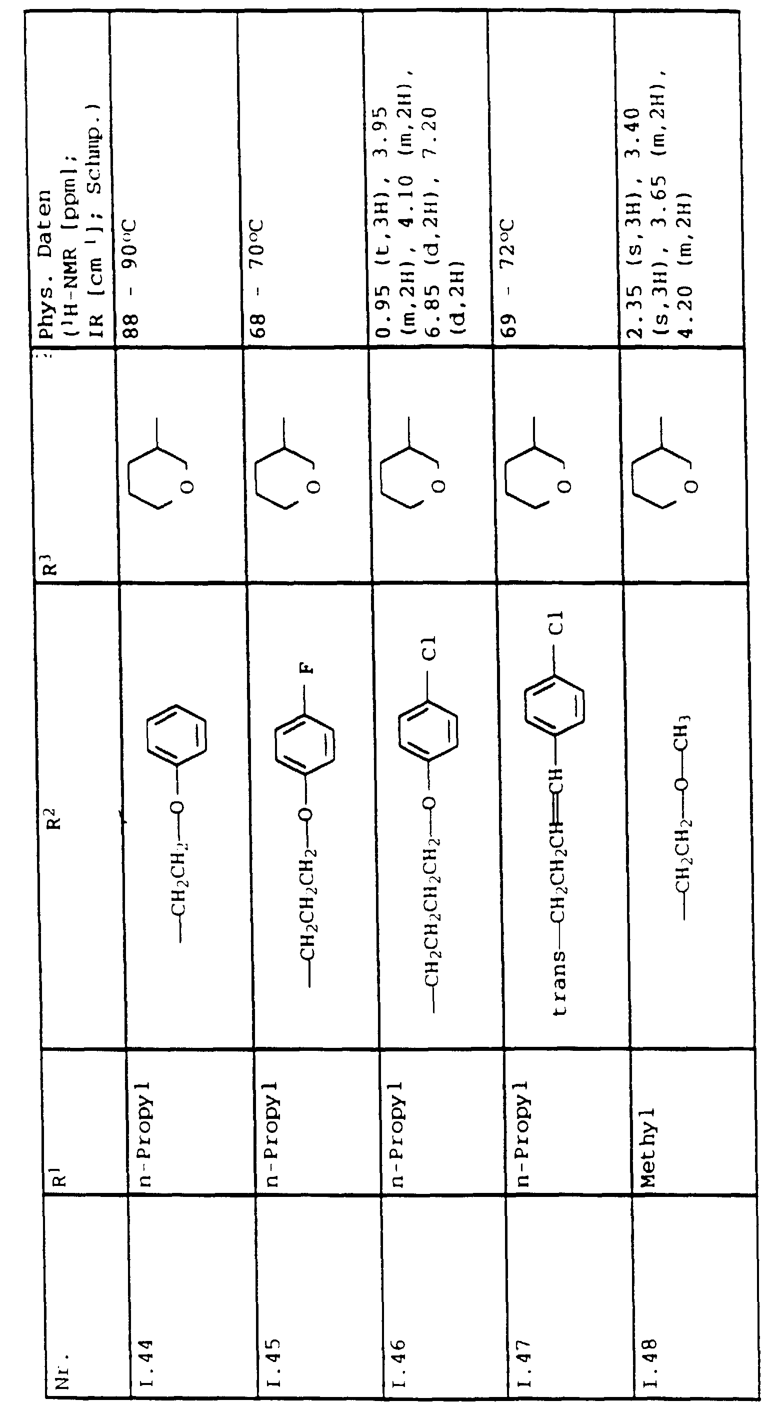

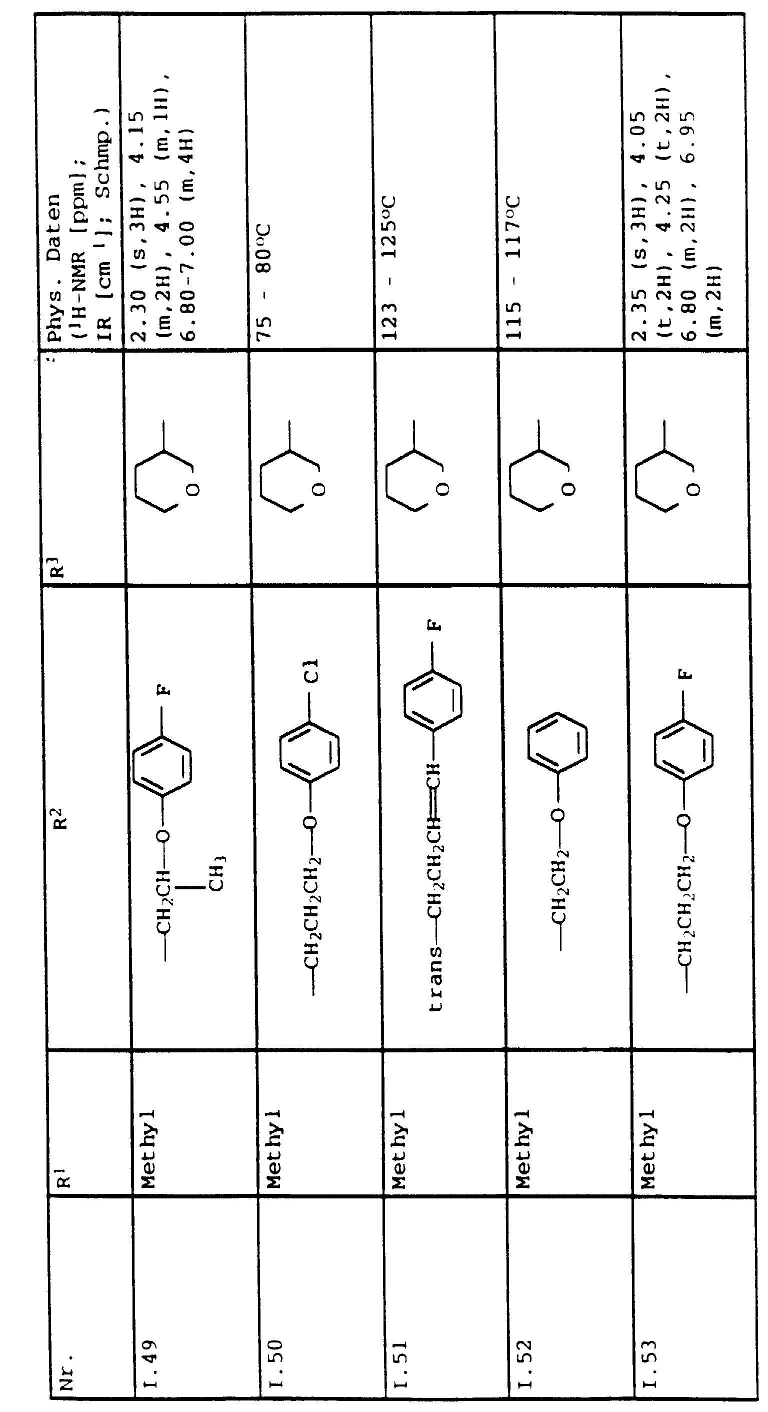

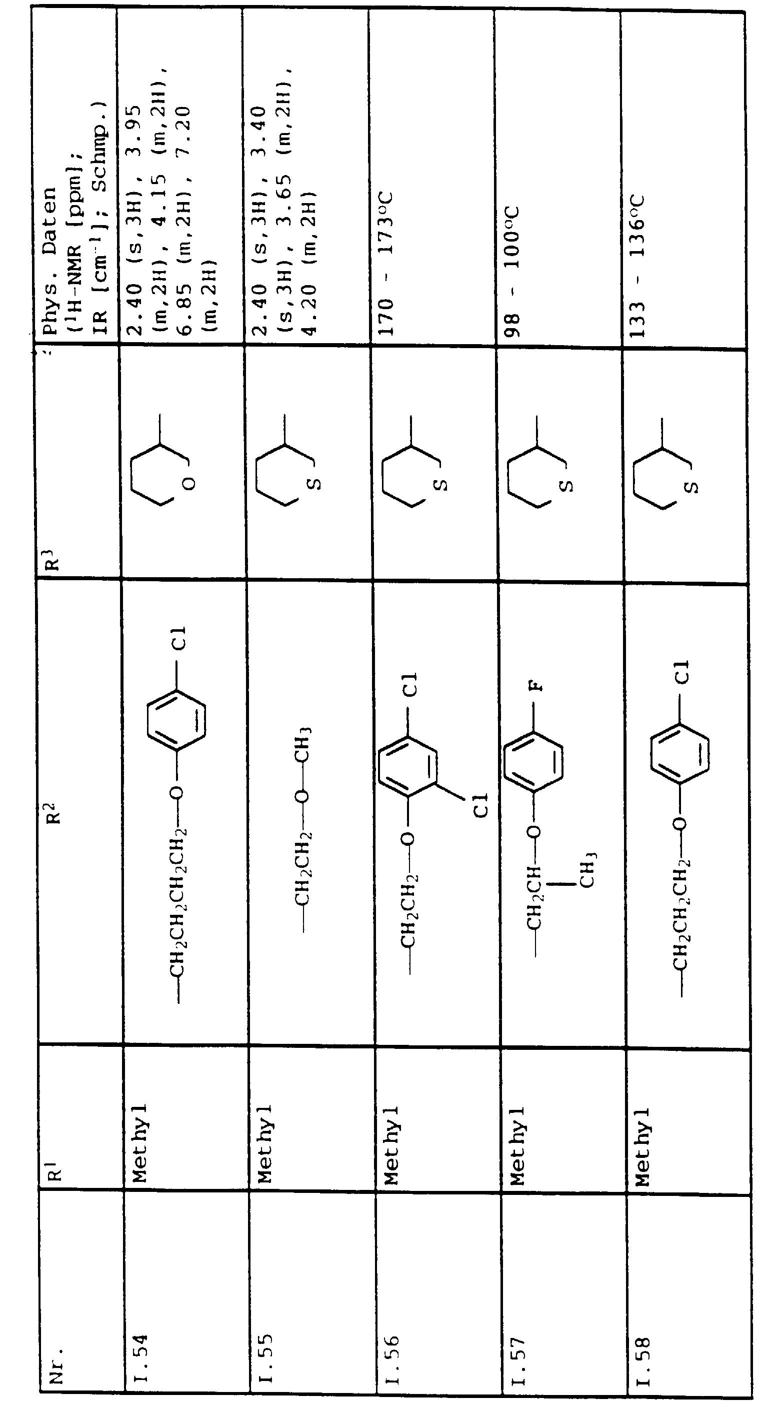

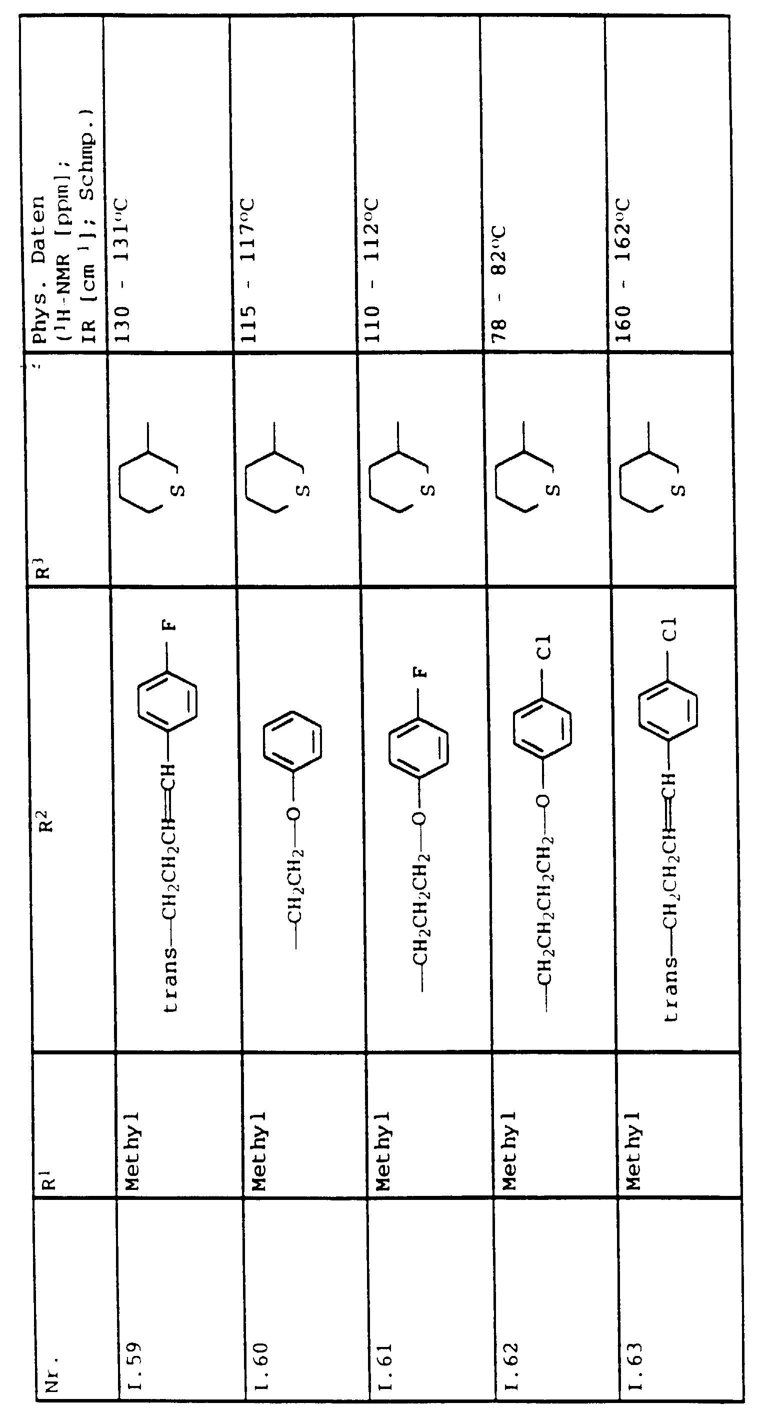

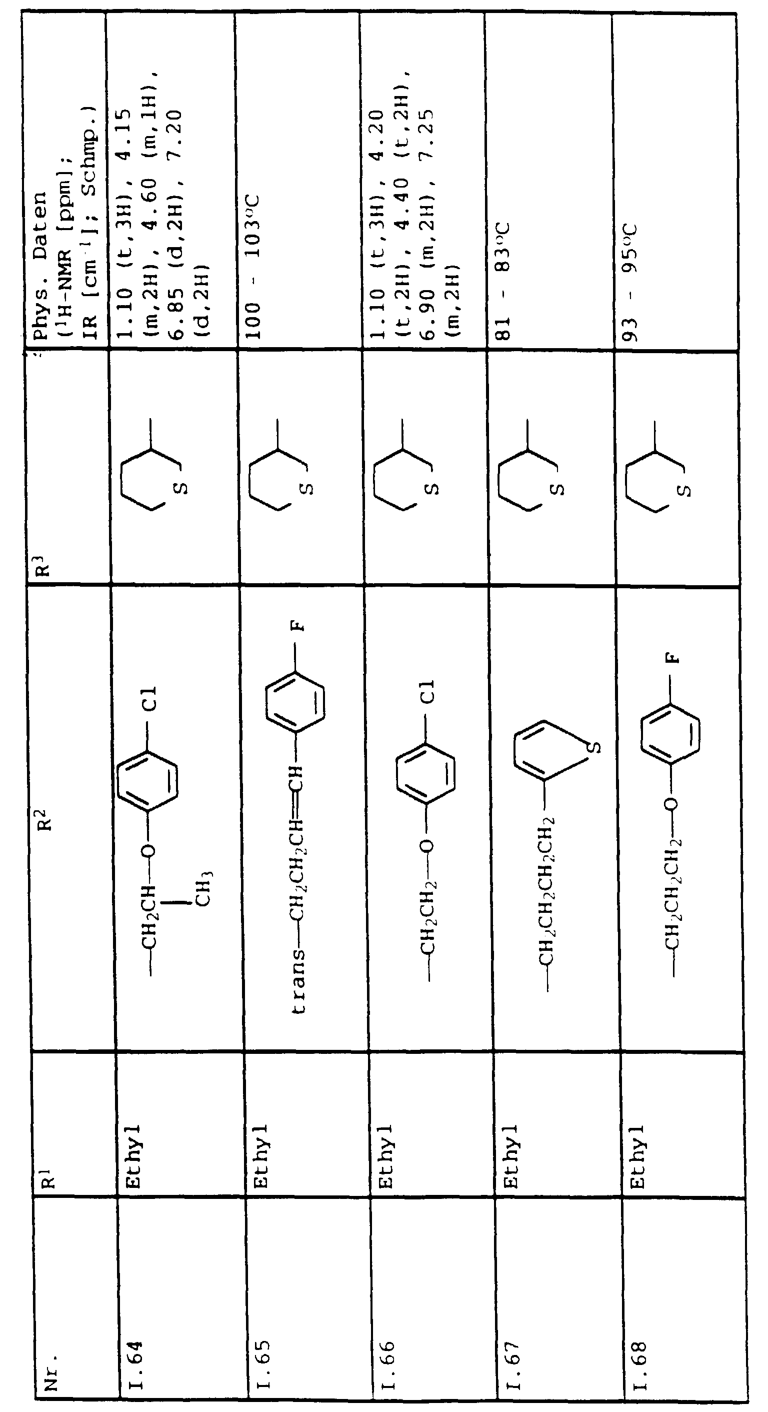

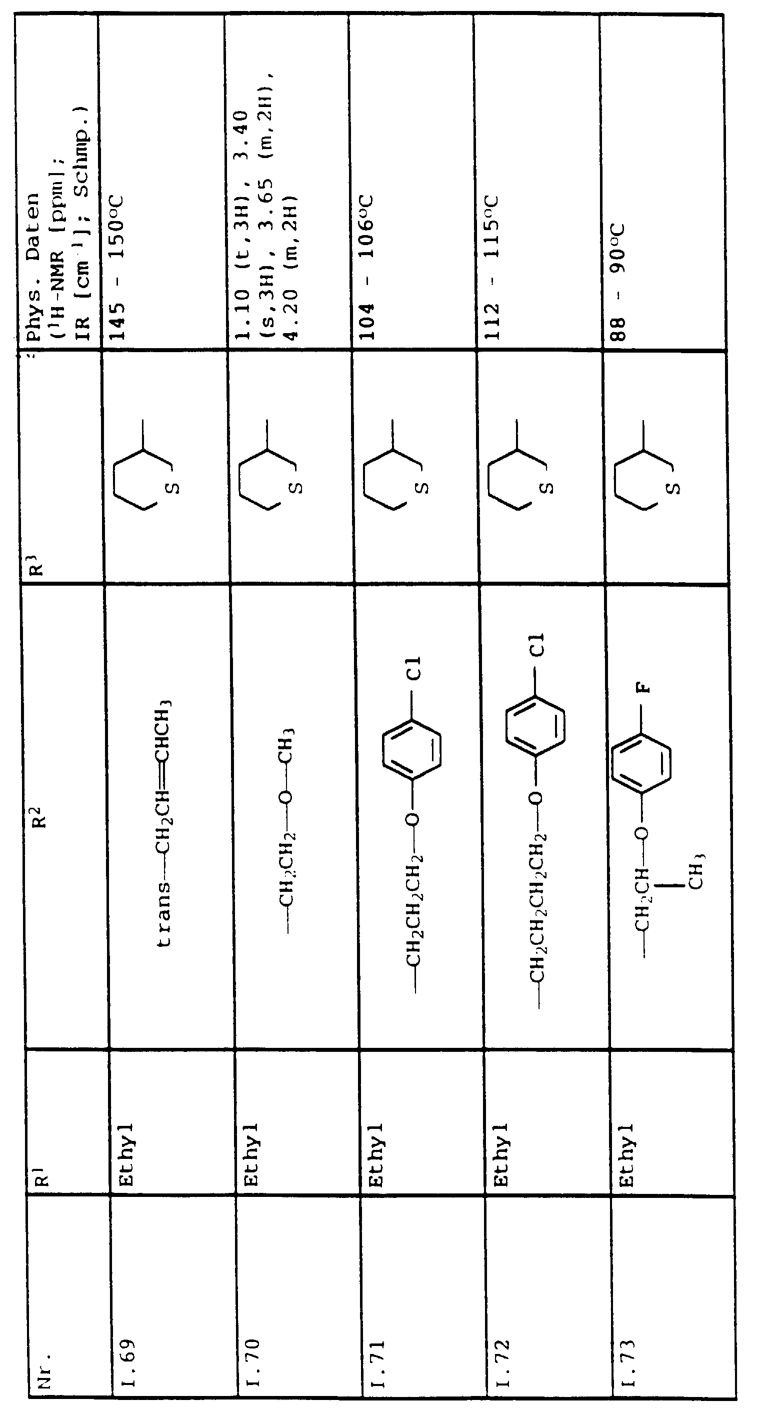

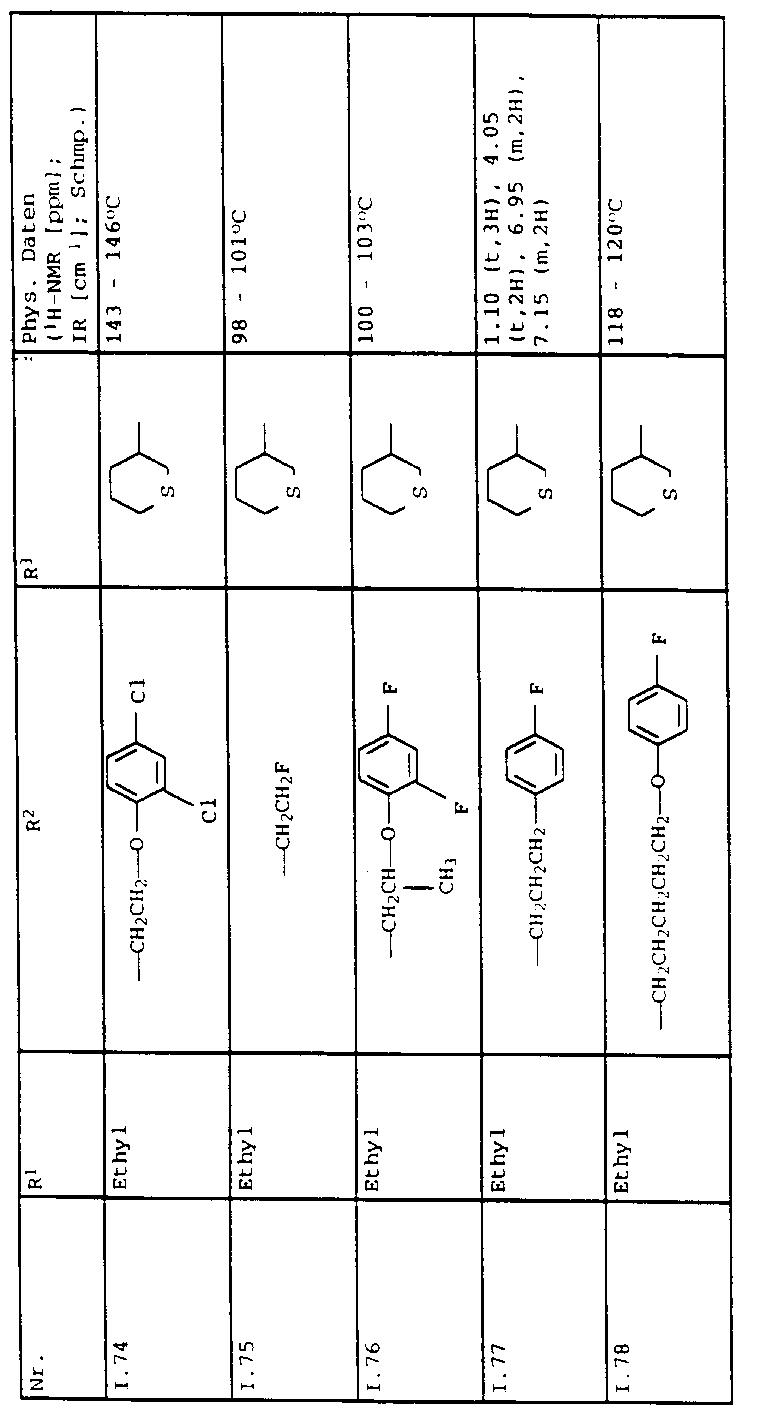

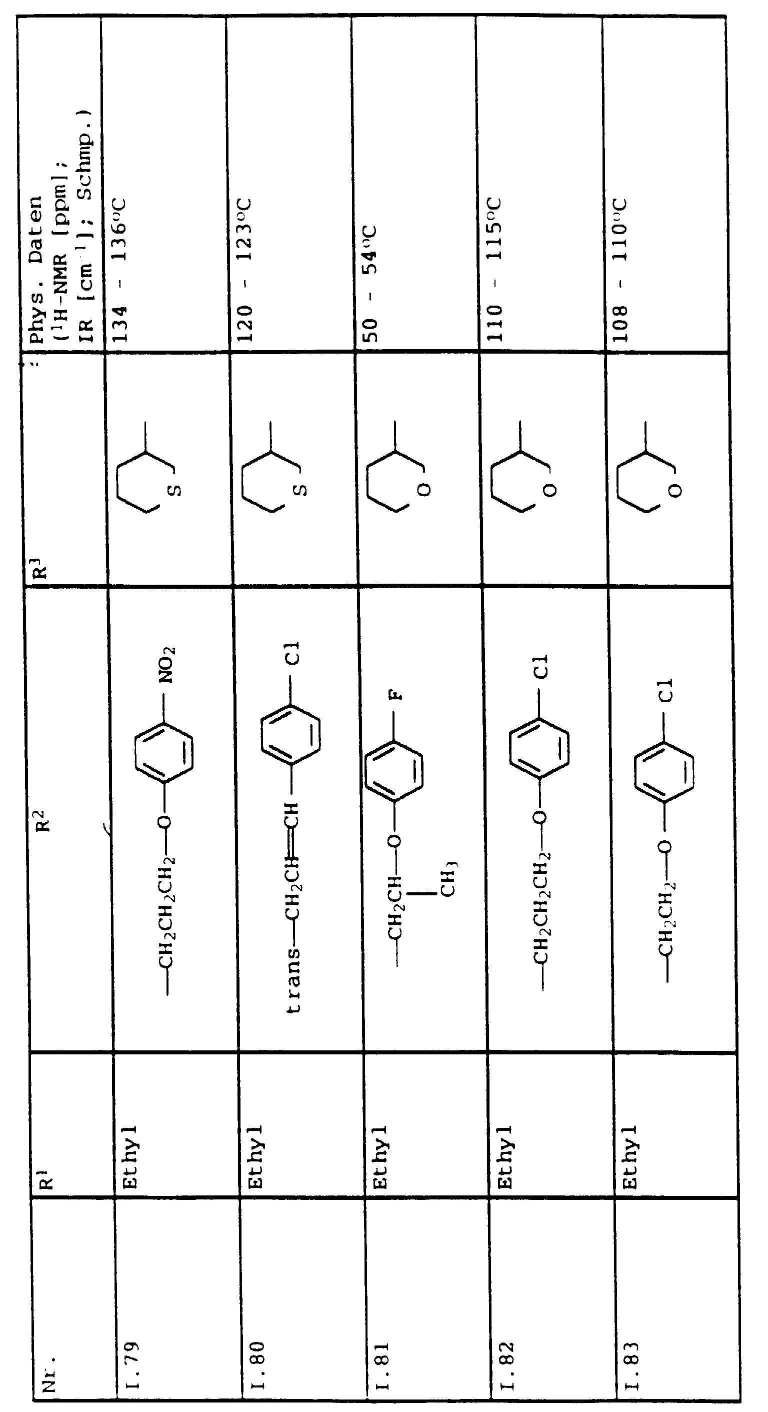

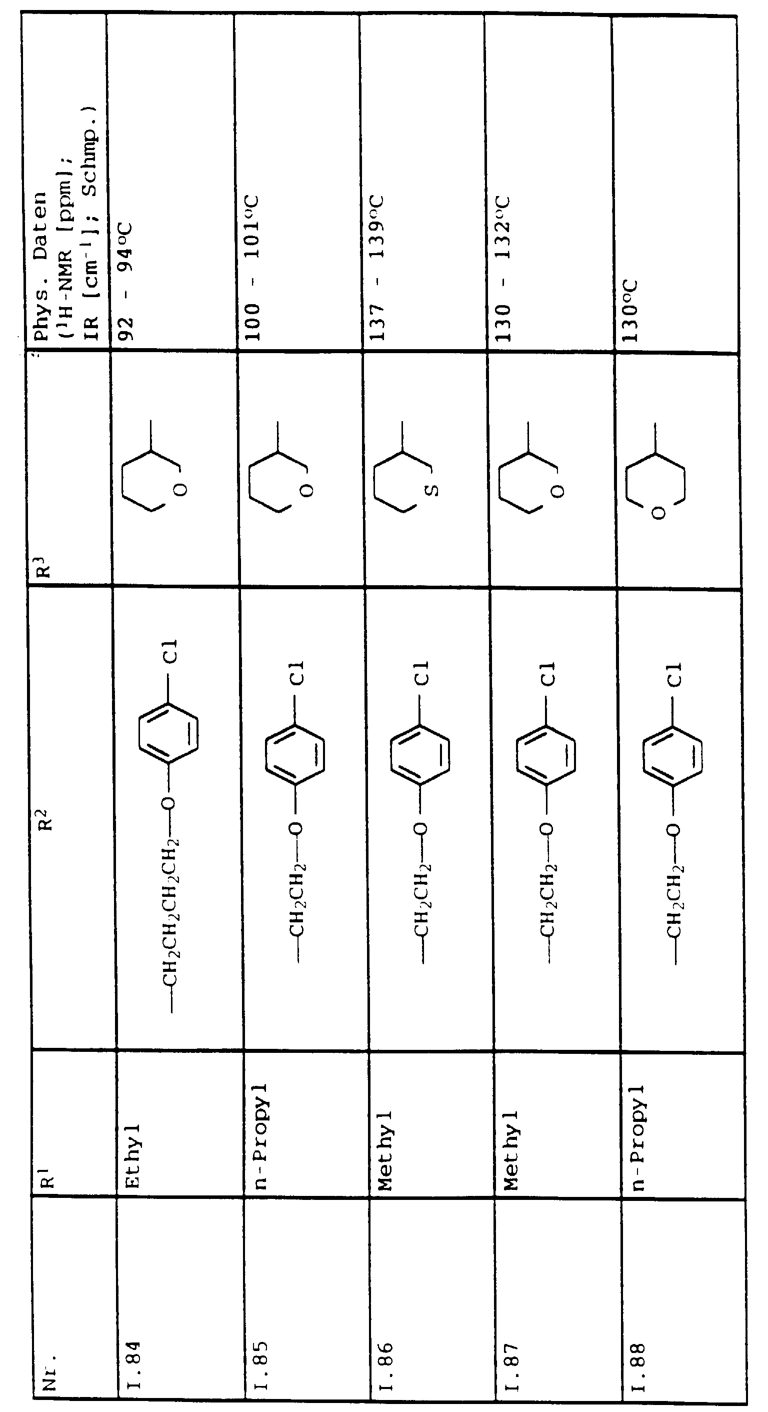

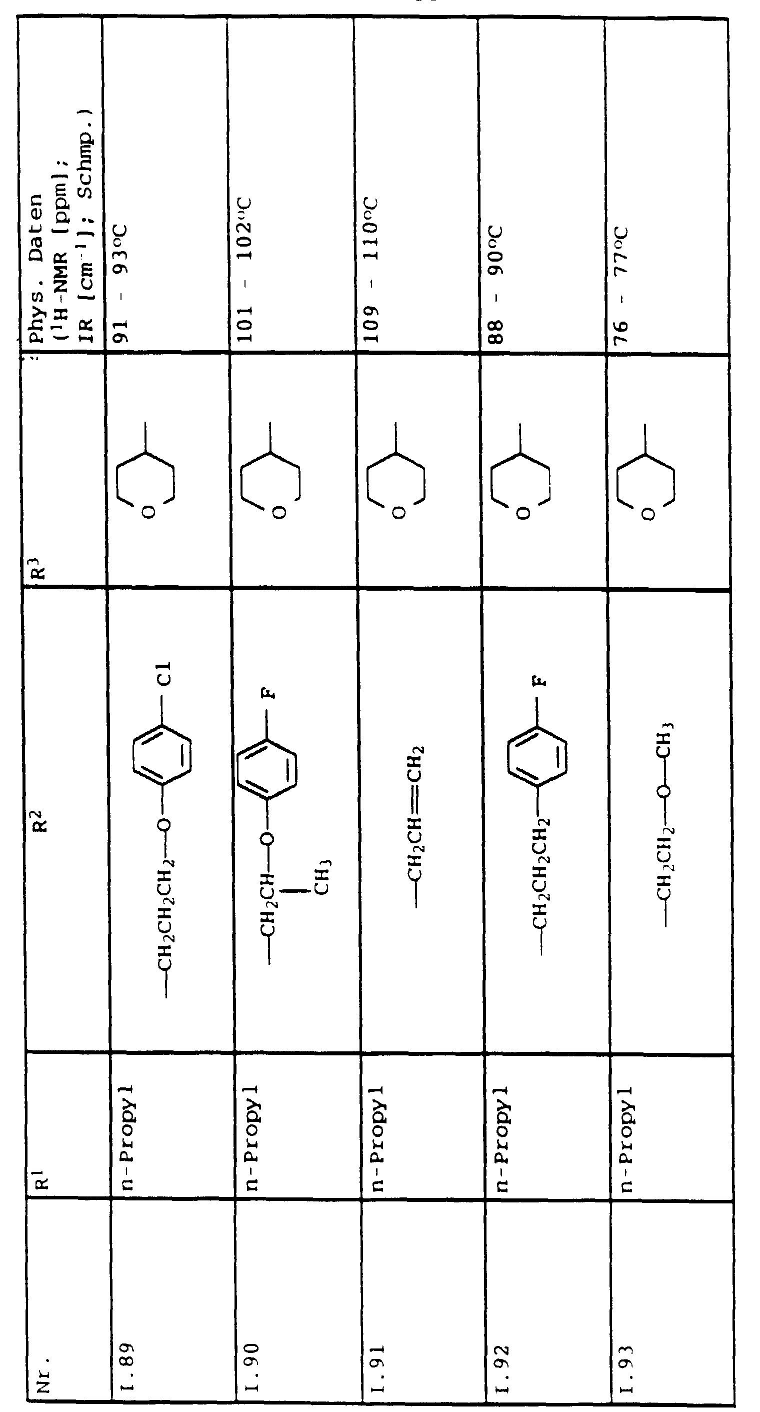

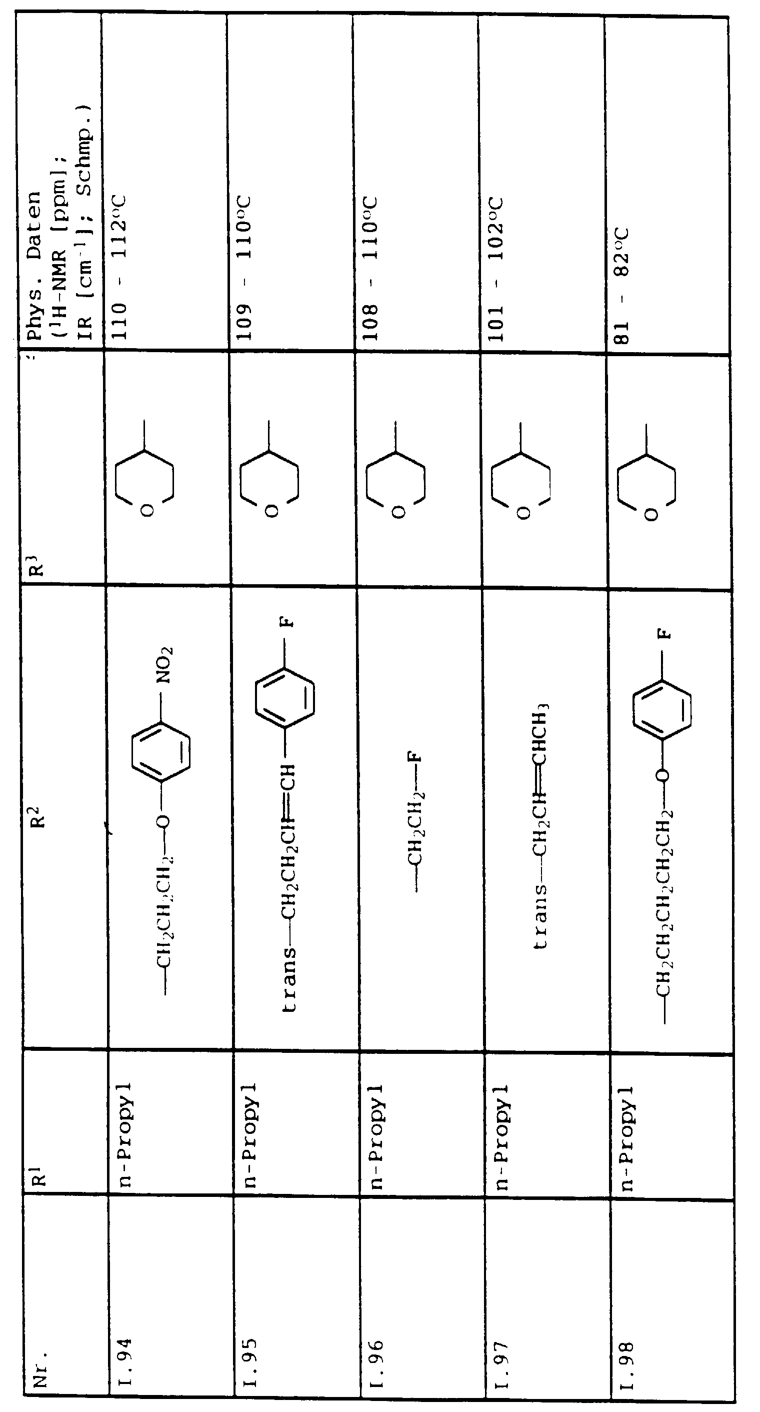

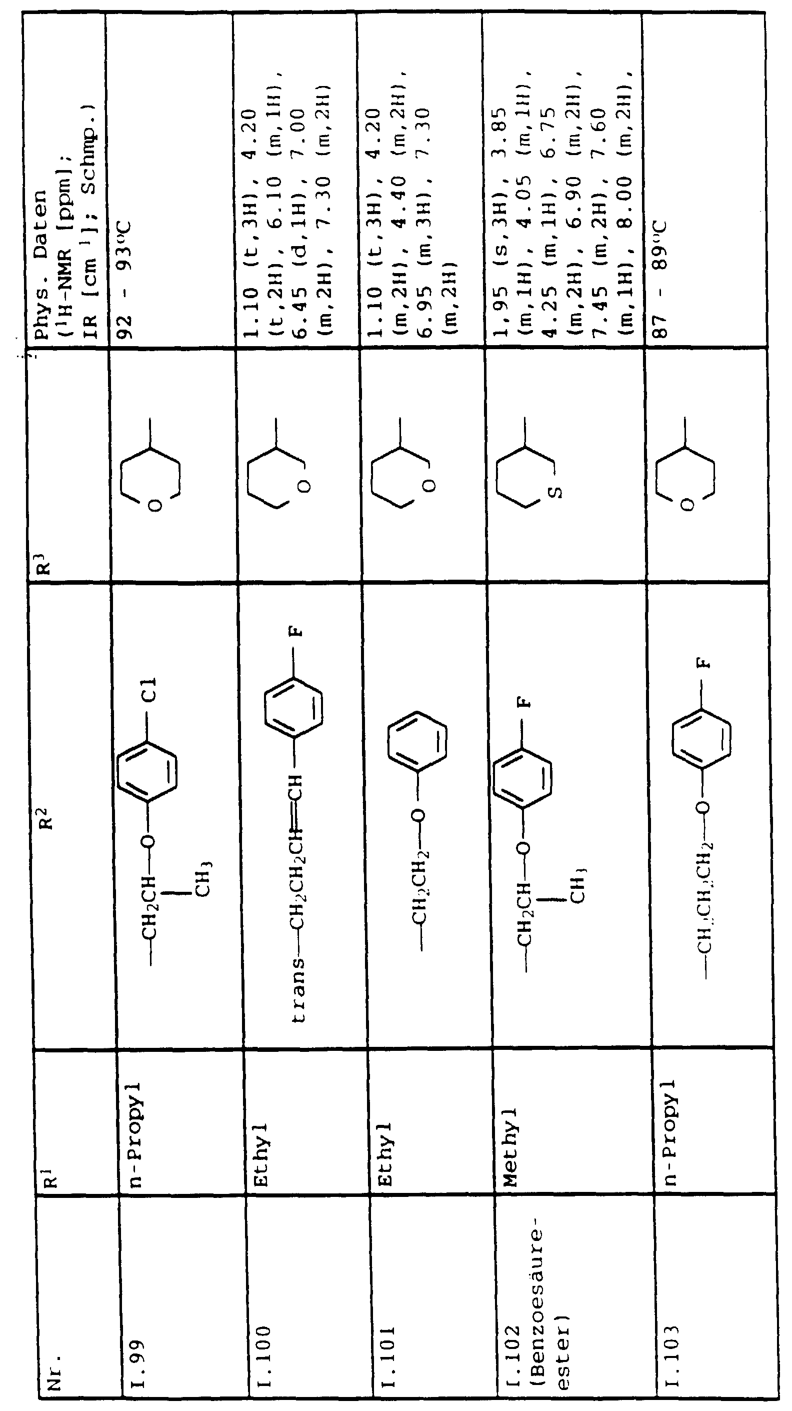

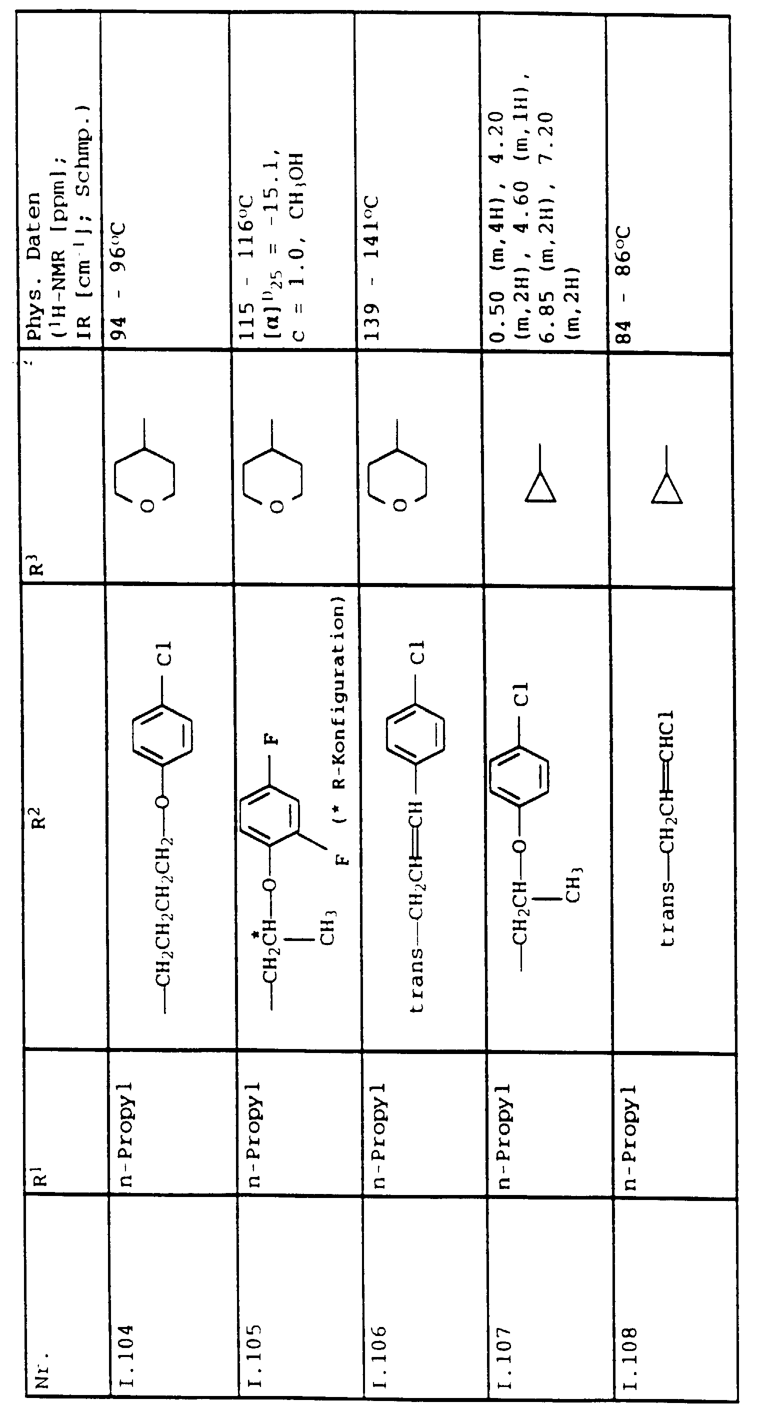

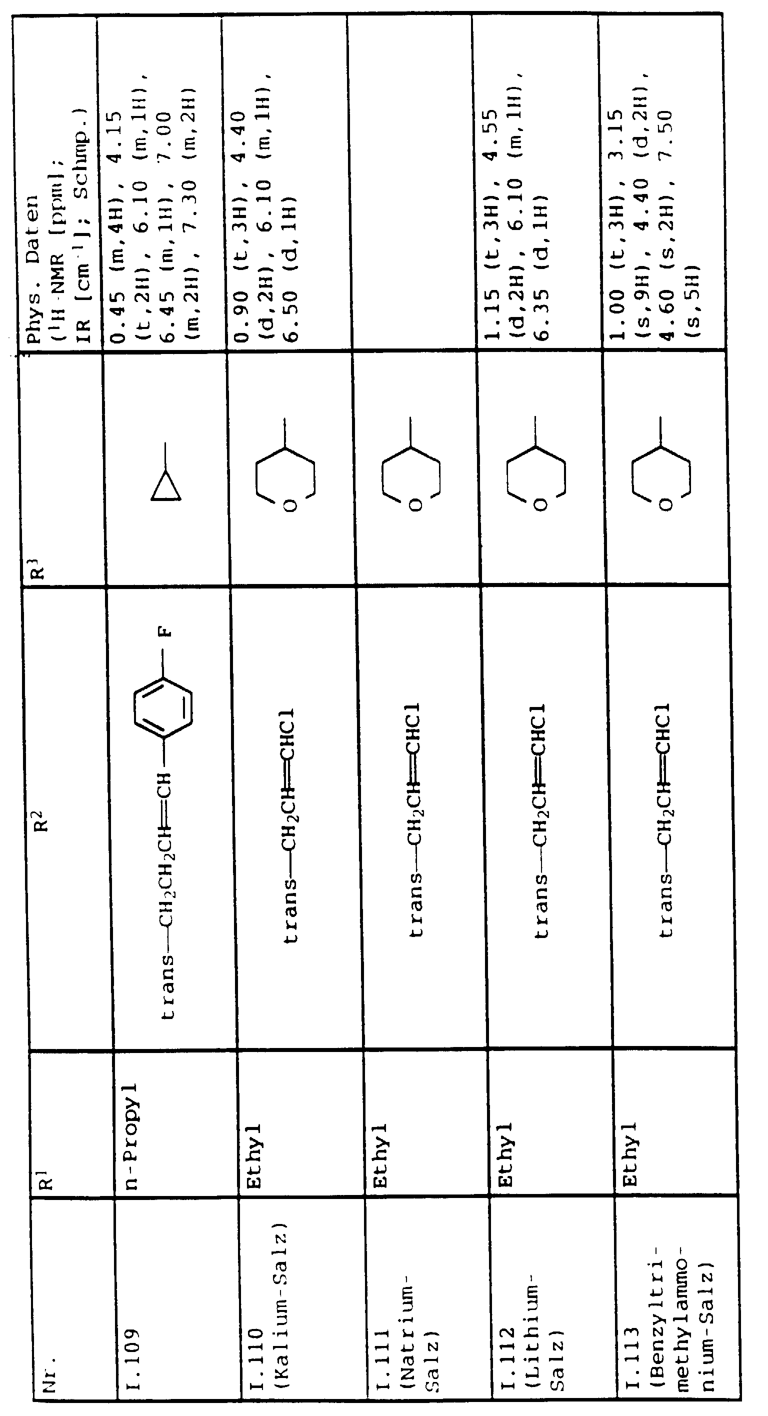

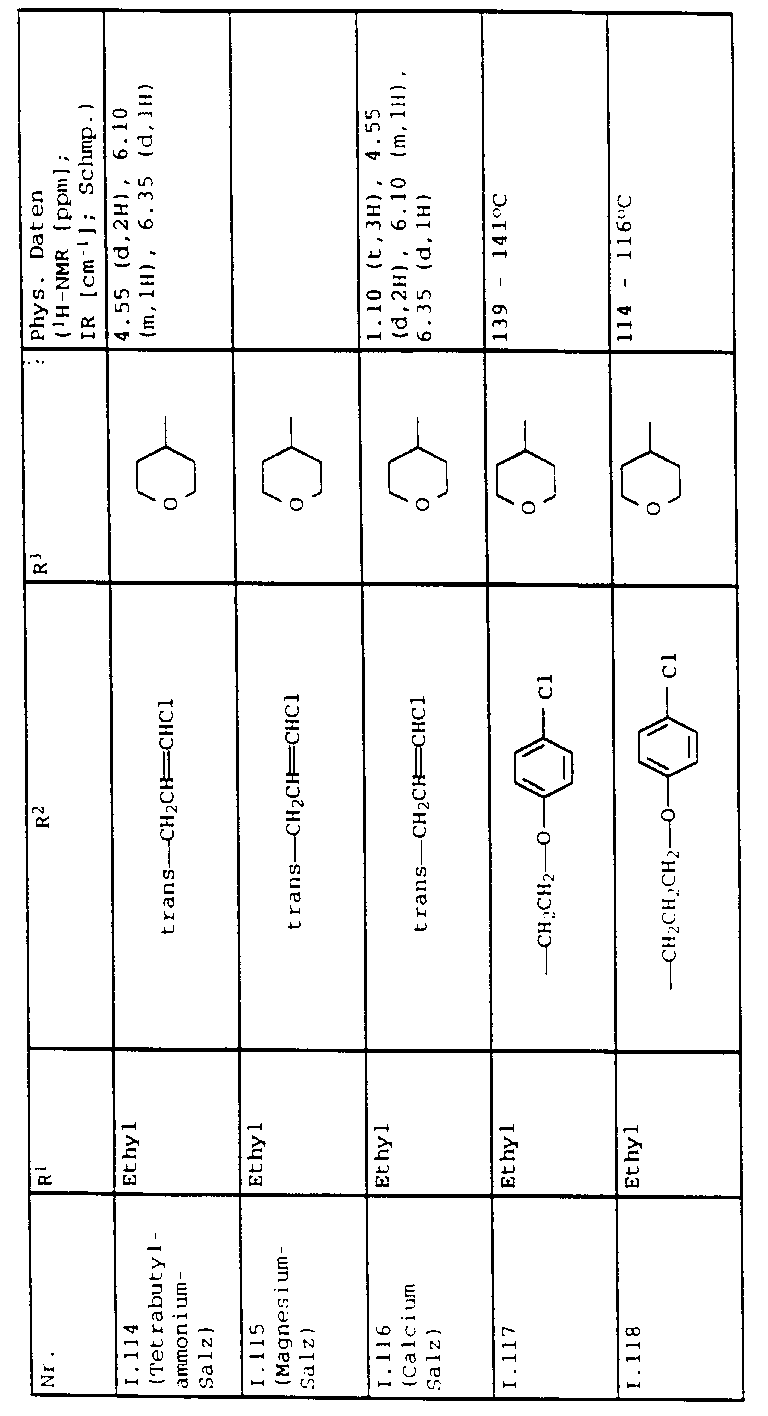

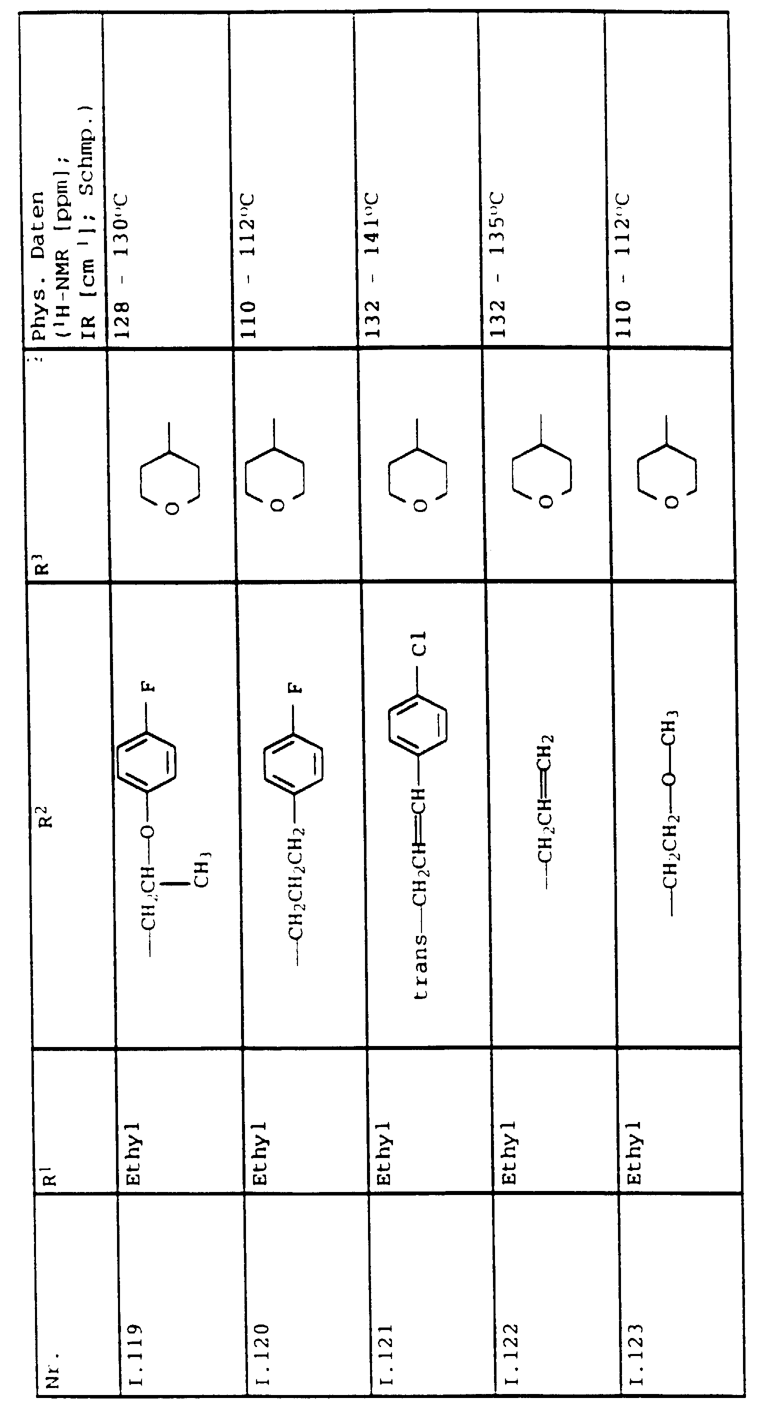

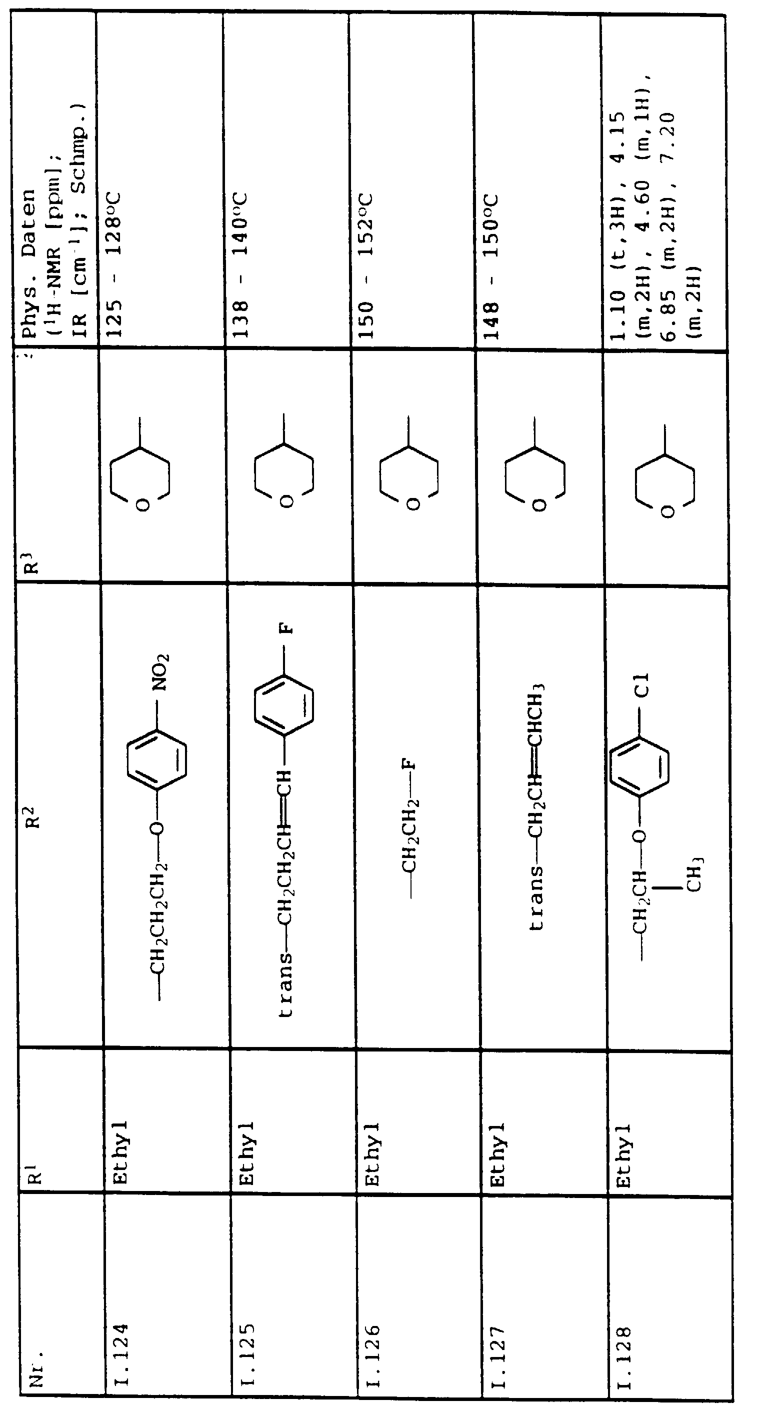

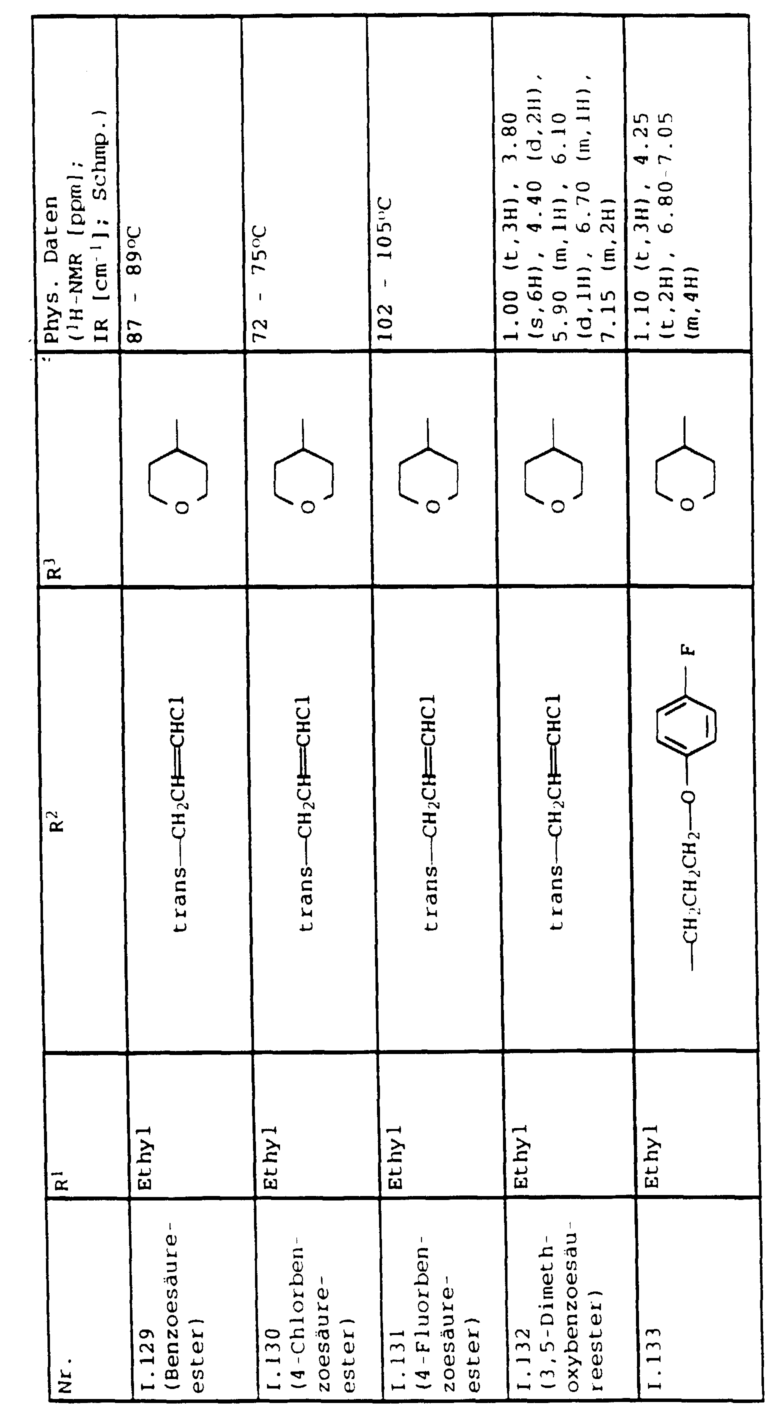

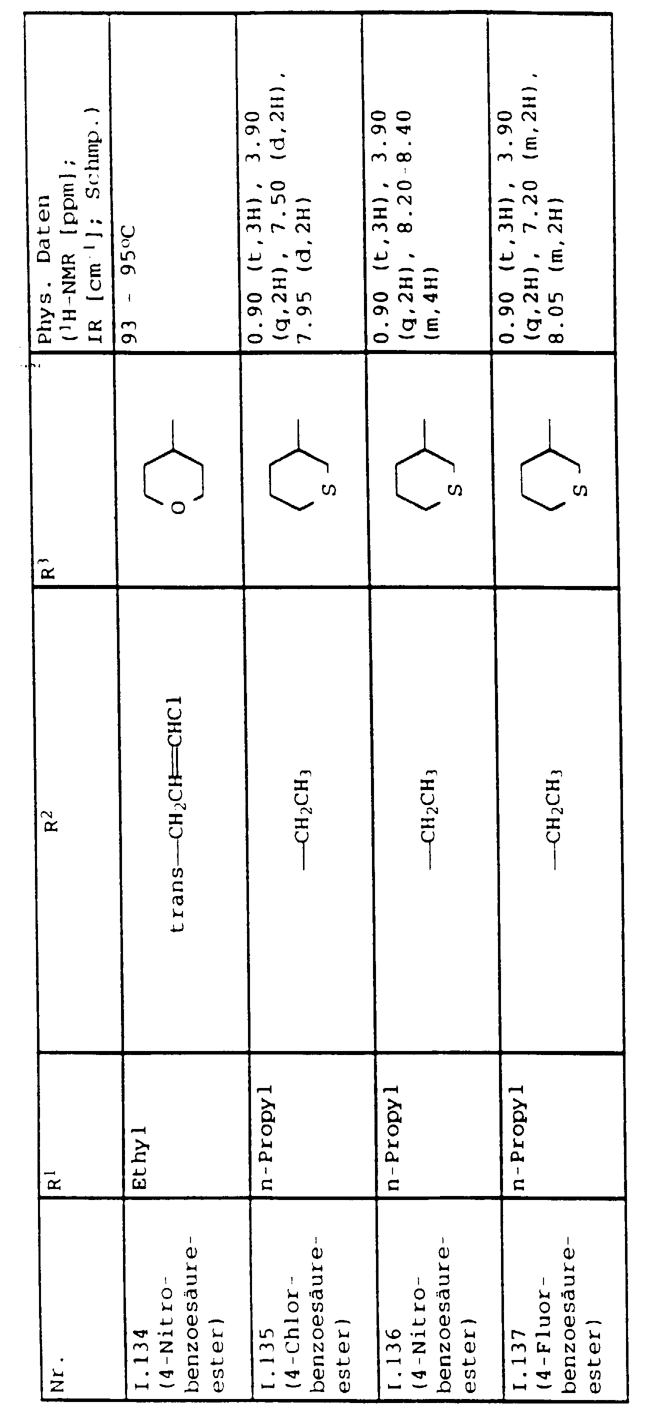

### Anwendungsbeispiele (herbizide Wirksamkeit)

Die herbizide Wirkung der Cyclohexenonoximether I ließ sich durch Gewachshausversuche zeigen:

Als Kulturgefäße dienten Plastikblumentöpfe mit lehmigem Sand mit etwa 3,0 Gew.-% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkte ein gleichmäßigeres Keimen der Testpflanzen, sofern dies nicht durch die Wirkstoffe beeinträchtigt wurde.

Zum Zwecke der Nachauflaufbehandlung wurden die Testpflanzen je nach Wuchsform bis zu einer Wuchshöhe von 3 bis 15 cm angezogen und erst dann mit den in Wasser suspendierten oder emulgierten Wirkstoffen behandelt. Die Testpflanzen wurden entweder bereits in den Versuchsgefäßen gesat und aufgezogen, in denen sie behandelt wurden, oder sie wurden als Keimpflanzen getrennt angezogen und einige Tage vor der Behandlung mit den Wirkstoffaufbereitungen in die Versuchsgefaße verpflanzt.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Als Vergleichsmittel diente die aus JP-A 1979/019,945 bekannte Verbindung A:

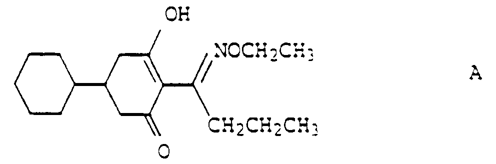

Die Aufwandmenge für die Nachauflaufbehandlung betrug 0,125 und 0,06 kg/ha a.S. (aktive Substanz).

Die in den folgenden Gewächshausverfahren verwendeten Pflanzen setzten sich aus folgenden Arten zusammen:

| Lateinischer Name | Deutscher Name |
|---|---|
| Avena fatua | Flughafer |
| Bromus inermis | unbegrannte Trespe |
| Digitaria sanguinalis | Blutfingerhirse |
| Echinochloa crus-galli | Hühnerhirse |
| Setaria italica | Ital. Raygrass |
| Sorghum halpense | Wilde Morgenhirse |
| Triticum aestivum | Winterweizen |

Das Ergebnis zeigte, daß die Verbindung Nr. I.27 unerwünschte Unkräuter besser bekampft und gleichzeitig verträglicher für die Kulturpflanze ist als der bekannte Wirkstoff A.

## Patentansprüche

1. Cyclohexenonoximether der Formel I 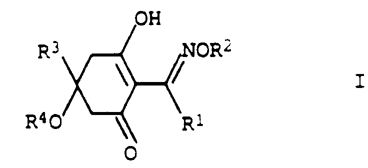 in der die Substituenten die folgende Bedeutung haben:
R¹ C₁-C₆-Alkyl;
R² C₁-C₁₀-Alkyl, C₁-C₁₀-Halogenalkyl, C₃-C₁₀-Alkenyl, C₃-C₁₀-Halogenalkenyl, C₃-C₁₀-Alkinyl oder C₃-C₁₀-Halogenalkinyl; -A¹-O-N=CH-Ph oder -A²-W, wobei
A¹ für C₂-C₄-Alkylen steht, welches ein bis drei C₁-C₃-Alkylgruppen tragen kann;
Ph für Phenyl steht, welches unsubstituiert sein oder ein bis drei Reste tragen kann, ausgewählt aus einer Gruppe bestehend aus Nitro, Cyano, Halogen, C₁-C₄-Alkyl und C₁-C₄-Halogenalkyl;
A² für C₁-C₆-Alkylen, C₃-C₆-Alkenylen oder C₃-C₆-Alkinylen steht, wobei diese Reste jeweils ein bis drei der folgenden Gruppen tragen können: Halogen und/oder C₁-C₃-Alkyl; oder
für C₂-C₅-Alkylenoxy, C₂-C₅-Alkenylenoxy oder C₂-C₄-Alkylenoxy-C₁-C₃-alkylen mit insgesamt drei bis fünf Kohlenstoffatomen steht, wobei diese Reste jeweils ein bis drei C₁-C₃-Alkylgruppen tragen können;
W für Phenyl, Pyridyl oder Thienyl steht, wobei die Ringe jeweils unsubstituiert sein oder ein bis drei der folgenden Reste tragen können: Nitro, Cyano, Halogen, C₁-C₄-Alkyl und/oder C₁-C₄-Halogenalkyl;
R³ C₁-C₆-Alkyl, welches eine der folgenden Gruppen trägt: C₁-C₄-Alkoxy oder C₁-C₄-Alkylthio; C₃-C₇-Cycloalkyl oder C₅-C₇-Cycloalkenyl, wobei diese Reste unsubstituiert sein oder ein bis drei der folgenden Substituenten tragen können: Hydroxy, Halogen, C₁-C₄-Alkyl, C₁-C₄-Halogenalkyl, C₁-C₄-Alkoxy und/oder C₁-C₄-Alkylthio; ein 5-gliedriger, gesättigter Heterocyclus, welcher neben Kohlenstoffringgliedern ein oder zwei Sauerstoff- und/oder Schwefelatome enthalten kann, wobei dieser Ring unsubstituiert sein oder ein bis drei der folgenden Substituenten tragen kann: C₁-C₄-Alkyl, C₁-C₄-Halogenalkyl, C₁-C₄-Alkoxy und/oder C₁-C₄-Alkylthio;
ein 6- oder 7-gliedriger, gesättigter oder ein- oder zweifach ungesättigter Heterocyclus, welcher neben Kohlenstoffringgliedern ein oder zwei Sauerstoff- und/oder Schwefelatome enthalten kann, wobei dieser Ring unsubstituiert sein oder ein bis drei der folgenden Substituenten tragen kann: Hydroxy, Halogen, C₁-C₄-Alkyl, C₁-C₄-Halogenalkyl, C₁-C₄-Alkoxy und/oder C₁-C₄-Alkylthio;
ein 5-gliedriger Heteroaromat, welcher neben Kohlenstoffringgliedern ein oder zwei Stickstoffatome und ein Sauerstoff- oder ein Schwefelatom enthält, wobei dieser Ring unsubstituiert sein oder ein bis drei der folgenden Substituenten tragen kann: Cyano, Halogen, C₁-C₄-Alkyl, C₁-C₄-Halogenalkyl, C₁-C₄-Alkoxy, C₁-C₄-Alkylthio, C₂-C₆-Alkenyl, C₂-C₆-Alkinyl und/oder C₁-C₄-Alkoxy-C₁-C₄-alkyl;
Phenyl oder Pyridyl, wobei diese Ringe unsubstituiert sein oder ein bis drei der folgenden Substituenten tragen können: Nitro, Cyano, Halogen, C₁-C₄-Alkyl, C₁-C₄-Halogenalkyl, C₁-C₄-Alkoxy, C₁-C₄-Alkylthio, C₃-C₆-Alkenyloxy, C₃-C₆-Alkinyloxy und/oder NR^{a}R^{b}, wobei
R^{a} für Wasserstoff, C₁-C₄-Alkyl, C₃-C₆-Alkenyl oder C₃-C₆-Alkinyl steht und
R^{b} für einen der Reste R^{a} oder für C₁-C₆-Alkylcarbonyl oder Benzoyl steht, wobei der Phenylring seinerseits ein bis drei der folgenden Reste tragen kann: Nitro, Cyano, Halogen, C₁-C₄-Alkyl, C₁-C₄-Halogenalkyl, C₁-C₄-Alkoxy und/oder C₁-C₄-Alkylthio;
R⁴ Wasserstoff;
C₁-C₁₀-Alkyl, C₁-C₁₀-Halogenalkyl, C₃-C₁₀-Alkenyl, C₃-C₁₀-Halogenalkenyl, C₃-C₁₀-Alkinyl, C₃-C₁₀-Halogenalkinyl, C₁-C₁₀-Alkylcarbonyl oder C₁-C₁₀-Halogenalkylcarbonyl; Benzoyl, wobei der Phenylring unsubstituiert sein oder ein bis drei der folgenden Reste tragen kann: Nitro, Cyano, Halogen, C₁-C₄-Alkyl, C₁-C₄-Halogenalkyl, C₁-C₄-Alkoxy und/oder C₁-C₄-Alkylthio;
-S(=O)₂-R^{c}; -P(=O)(OR^{d})(OR^{e}) oder -SiR^{f}R^{g}R^{h}, wobei
R^{c}, R^{d}, R^{e}, R^{f}, R^{g} und R^{h} unabhängig voneinander für C₁-C₁₀-Alkyl, C₁-C₁₀-Halogenalkyl oder Phenyl stehen, wobei der Phenylring seinerseits ein bis drei der folgenden Reste tragen kann: Nitro, Cyano, Halogen, C₁-C₄-Alkyl, C₁-C₄-Halogenalkyl, C₁-C₄-Alkoxy und/oder C₁-C₄-Alkylthio;
wobei R³ nicht für 2-(Ethylthio)propyl steht, wenn R¹ Ethyl, R² 3-Chlor-2-propenyl und R⁴ Wasserstoff bedeuten,
sowie die landwirtschaftlich brauchbaren Salze von I und die Ester aus C₁-C₁₀-Carbonsäuren oder anorganischen Säuren und den Verbindungen I.

2. Verfahren zur Herstellung der Verbindungen der Formel Ia 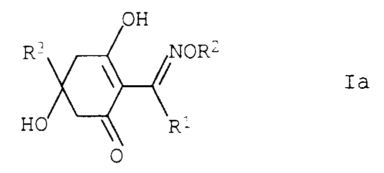 in der R¹, R² und R³ die in Anspruch 1 gegebene Bedeutung haben, dadurch gekennzeichnet, daß man ein Triketon der Formel II
(CH₃C=O)₂CH-CO-R¹ II
in an sich bekannter Weise in einem inerten organischen Lösungsmittel in Gegenwart einer Base mit einem Säurehalogenid der Formel III
R³-CO-Hal III
in der Hal für ein Halogenatom steht, zu einem Cyclohexenonketon der Formel IV 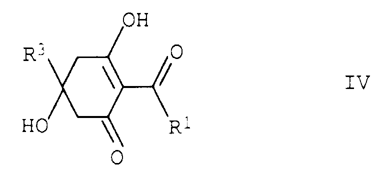 cyclisiert und IV anschließend in an sich bekannter Weise in einem inerten organischen Lösungsmittel mit einem Hydroxylamin der Formel V
H₂N-OR² V
oder einem entsprechenden Hydroxylammoniumsalz umsetzt.

3. Verfahren zur Herstellung der Verbindungen der Formel Ib 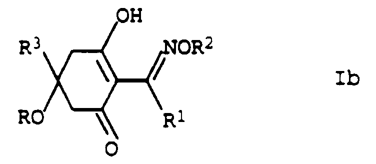 in der R¹, R² und R³ die in Anspruch 1 gegebene Bedeutung haben und R einen der Reste R⁴ gemäß Anspruch 1 ausgenommen Wasserstoff bedeutet, dadurch gekennzeichnet, daß man eine Verbindung Ia, gemäß Anspruch 2, in an sich bekannter Weise in einem inerten organischen Lösungsmittel mit einer Verbindung VI
R-X VI,
in der X für Halogen steht, umsetzt.

4. Herbizide Mittel enthaltend eine herbizid wirksame Menge mindestens eines Cyclohexenonoximethers der Formel I gemäß Anspruch 1, sowie inerte Zusatzstoffe.

5. Verfahren zur Bekämpfung unerwünschten Pflanzenwuchses, dadurch gekennzeichnet, daß man die Pflanzen und/oder ihren Lebensraum mit einer wirksamen Menge eines Cyclohexenonoximethers der Formel I gemäß Anspruch 1 behandelt.

6. Verwendung von Cyclohexenonoximethern der Formel I gemäß Anspruch 1 zur Bekämpfung von unerwünschtem Pflanzenwuchs.

7. Cyclohexenonketone der Formel IV, gemäß Anspruch 2.

8. Verwendung von Cyclohexenonketonen der Formel IV gemaß Anspruch 2 als Zwischenprodukte.

## Claims

1. A cyclohexenone oxime ether of the formula I 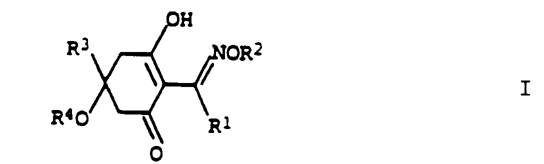 where
R¹ is C₁-C₆-alkyl;
R² is C₁-C₁₀-alkyl, C₁-C₁₀-haloalkyl, C₃-C₁₀-alkenyl, C₃-C₁₀-haloalkenyl, C₃-C₁₀-alkynyl or C₃-C₁₀-haloalkynyl; -A¹-O-N=CH-Ph or -A²-W;
A¹ is C₂-C₄-alkylene which may carry from one to three C₁-C₃-alkyl groups;
Ph is phenyl which may be unsubstituted or may carry from one to three radicals selected from the group consisting of nitro, cyano, halogen, C₁-C₄-alkyl and C₁-C₄-haloalkyl;
A² is C₁-C₆-alkylene, C₃-C₆-alkenylene or C₃-C₆-alkynylene, where these radicals may in each case carry from one to three of the following groups: halogen and/or C₁-C₃-alkyl;
or
C₂-C₅-alkyleneoxy, C₂-C₅-alkenyleneoxy or C₂-C₄-alkyleneoxy-C₁-C₃-alkylene having a total of from three to five carbon atoms, where these radicals may in each case carry from one to three C₁-C₃-alkyl groups;
W is phenyl, pyridyl or thienyl, where the rings may in each case be unsubstituted or may carry from one to three of the following radicals: nitro, cyano, halogen, C₁-C₄-alkyl and/or C₁-C₄-haloalkyl;
R³ is C₁-C₆-alkyl which carries one of the following groups: C₁-C₄-alkoxy or C₁-C₄-alkylthio;
C₃-C₇-cycloalkyl or C₅-C₇-cycloalkenyl, where these radicals may be unsubstituted or may carry from one to three of the following substituents: hydroxyl, halogen, C₁-C₄-alkyl, C₁-C₄-haloalkyl, C₁-C₄-alkoxy and/or C₁-C₄-alkylthio;
a 5-membered, saturated heterocyclic structure which, in addition to carbon ring members, may contain one or two oxygen and/or sulfur atoms, where this ring may be unsubstituted or may carry from one to three of the following substituents: C₁-C₄-alkyl, C₁-C₄-haloalkyl, C₁-C₄-alkoxy and/or C₁-C₄-alkylthio;
a 6-membered or 7-membered, saturated or mono- or di-unsaturated heterocyclic structure which, in addition to carbon ring members, may contain one or two oxygen and/or sulfur atoms, where this ring may be unsubstituted or may carry from one to three of the following substituents: hydroxyl, halogen, C₁-C₄-alkyl, C₁-C₄-haloalkyl, C₁-C₄-alkoxy or C₁-C₄-alkylthio;
a 5-membered heteroaromatic structure which, in addition to carbon ring members, contains one or two nitrogen atoms and one oxygen or one sulfur atom, where this ring may be unsubstituted or may carry from one to three of the following substituents: cyano, halogen, C₁-C₄-alkyl, C₁-C₄-haloalkyl, C₁-C₄-alkoxy, C₁-C₄-alkylthio, C₂-C₆-alkenyl, C₂-C₆-alkynyl and/or C₁-C₄-alkoxy-C₁-C₄-alkyl;
phenyl or pyridyl, where these rings may be unsubstituted or may carry from one to three of the following substituents: nitro, cyano, halogen, C₁-C₄-alkyl, C₁-C₄-haloalkyl, C₁-C₄-alkoxy, C₁-C₄-alkylthio, C₃-C₆-alkenyloxy, C₃-C₆-alkynyloxy and/or NR^{a}R^{b},
R^{a} is hydrogen, C₁-C₄-alkyl, C₃-C₆-alkenyl or C₃-C₆-alkynyl, R^{b} is one of the radicals R^{a} or is C₁-C₆-alkylcarbonyl or benzoyl, where the phenyl ring in turn may carry from one to three of the following radicals: nitro, cyano, halogen, C₁-C₄-alkyl, C₁-C₄-haloalkyl, C₁-C₄-alkoxy and/or C₁-C₄-alkylthio;
R⁴ is hydrogen;
C₁-C₁₀-alkyl, C₁-C₁₀-haloalkyl, C₃-C₁₀-alkenyl, C₃-C₁₀-haloalkenyl, C₃-C₁₀-alkynyl, C₃-C₁₀-haloalkynyl, C₁-C₁₀-alkylcarbonyl or C₁-C₁₀-haloalkylcarbonyl;
benzoyl, where the phenyl ring may be unsubstituted or may carry from one to three of the following radicals: nitro, cyano, halogen, C₁-C₄-alkyl, C₁-C₄-haloalkyl, C₁-C₄-alkoxy and/or C₁-C₄-alkylthio;
-S(=O)₂-R^{c}, -P(=O)(OR^{d})(OR^{e}) or -SiR^{f}R^{g}R^{h}, and
R^{c}, R^{d}, R^{e}, R^{f}, R^{g} and R^{h} independently of one another are each C₁-C₁₀-alkyl, C₁-C₁₀-haloalkyl or phenyl, where the phenyl ring in turn may carry from one to three of the following radicals: nitro, cyano, halogen, C₁-C₄-alkyl, C₁-C₄-haloalkyl, C₁-C₄-alkoxy and/or C₁-C₄-alkylthio, and R³ is not 2-(ethylthio)propyl when R¹ is ethyl, R² is 3-chloro-2-propenyl and R⁴ is hydrogen,
and the agriculturally useful salts of I and the esters of C₁-C₁₀-carboxylic acids or inorganic acids and the compounds I.

2. A process for the preparation of a compound of the formula Ia 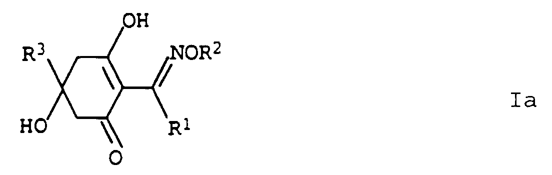 where R¹, R² and R³ have the meanings stated in claim 1, wherein a triketone of the formula II
(CH₃C=O)₂CH-CO-R¹ II
is cyclized in a conventional manner, in an inert organic solvent in the presence of a base, with an acyl halide of the formula III
R³-CO-Hal III
where Hal is halogen, to give a cyclohexenone ketone of the formula IV 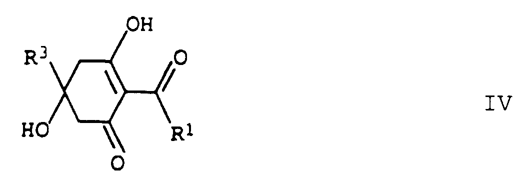 and IV is then reacted in a conventional manner, in an inert organic solvent, with a hydroxylamine of the formula V
H₂N-OR² V
or with a corresponding hydroxylammonium salt.

3. A process for the preparation of a compound of the formula Ib 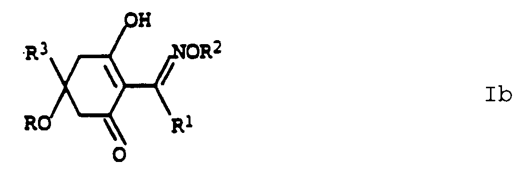 where R¹, R² and R³ have the meanings stated in claim 1 and R is one of the radicals R⁴ as claimed in claim 1, with the exception of hydrogen, wherein a compound Ia as claimed in claim 2 is reacted in a conventional manner, in an inert organic solvent, with a compound VI
R-X VI
where X is halogen.

4. A herbicide containing a herbicidally effective amount of at least one cyclohexenone oxime ether of the formula I as claimed in claim 1 and inert additives.

5. A method for controlling undesirable plant growth, wherein the plants and/or their habitat is or are treated with an effective amount of a cyclohexenone oxime ether of the formula I as claimed in claim 1.

6. Use of a cyclohexenone oxime ether of the formula I as claimed in claim 1 for controlling undesirable plant growth.

7. A cyclohexenone ketone of the formula IV as claimed in claim 2.

8. Use of a cyclohexenone ketone of the formula IV as claimed in claim 2 as an intermediate.

## Revendications

1. Ethers de cyclohexénonoximes de formule I 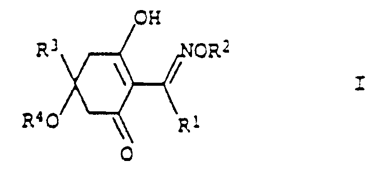 dans laquelle les symboles ont les significations suivantes :
R¹ un groupe alkyle en C1-C6 ;
R² un groupe alkyle en C1-C10, halogénoalkyle en C1-C10, alcényle en C3-C10, halogénoalcényle en C3-C10, alcynyle en C3-C10 ou halogénoalcynyle en C3-C10 ;
-A¹-O-N=CH-Ph ou -A²-W,
A¹ représentant un groupe alkylène en C2-C4 qui peut porter un à trois groupes alkyle en C1-C3 ;
Ph représentant un groupe phényle non substitué ou portant un à trois substituants choisis parmi les groupes nitro, cyano, les halogènes, les groupes alkyle en C1-C4 et halogénoalkyle en C1-C4 ;
A² représentant un groupe alkylène en C1-C6, alcénylène en C3-C6 ou alcynylène en C3-C6, chacun de ces groupes pouvant porter un à trois des substituants suivants : halogéno et/ou alkyle en C1-C3 ;
ou bien
un groupe alkylénoxy en C2-C5, alcénylénoxy en C2-C5 ou (alkylène en C2-C4)oxy-alkylène en C1-C3 contenant au total trois à cinq atomes de carbone, chacun de ces groupes pouvant porter un à trois groupes alkyle en C1-C3 ;
W représente un groupe phényle, pyridyle ou thiényle, dont les cycles sont non substitués ou portent un à trois des substituants suivants : nitro, cyano, halogéno, alkyle en C1-C4 et/ou halogénoalkyle en C1-C4;
R³ un groupe alkyle en C1-C6 qui porte un des substituants suivants :
alcoxy en C1-C4 ou alkylthio en C1-C4 ;
cycloalkyle en C3-C7 ou cycloalcényle en C5-C7, ces groupes pouvant être non substitués ou pouvant porter un à trois des substituants suivants : hydroxy, halogéno, alkyle en C1-C4, halogénoalkyle en C1-C4, alcoxy en C1-C4 et/ou alkylthio en C1-C4 ;
un hétérocycle saturé à cinq chaînons qui, avec des chaînes cycliques carbonées, peut contenir un ou deux atomes d'oxygène et/ou de soufre, ce cycle étant non substitué ou portant un à trois des substituants suivants : alkyle en C1-C4, halogénoalkyle en C1-C4, alcoxy en C1-C4 et/ou alkylthio en C1-C4 ;
un hétérocycle saturé ou mono- ou di-insaturé à six ou sept chaînons qui, avec les chaînons cycliques carbonés, peut contenir un ou deux atomes d'oxygène et/ou de soufre, ce cycle étant non substitué ou portant un à trois des substituants suivants : hydroxy, halogéno, alkyle en C1-C4, halogénoalkyle en C1-C4, alcoxy en C1-C4 et/ou alkylthio en C1-C4 ;
un groupe hétéroaromatique à cinq chaînons qui, avec les chaînons cycliques carbonés, contient un ou deux atomes d'azote et un atome d'oxygène ou de soufre, ce cycle étant non substitué ou portant un à trois des substituants suivants : cyano, halogéno, alkyle en C1-C4, halogénoalkyle en C1-C4, alcoxy en C1-C4, alkylthio en C1-C4, alcényle en C2-C6, alcynyle en C2-C6 et/ou (alcoxy en C1-C4)-alkyle en C1-C4 ;
un groupe phényle ou pyridyle, chacun d'eux pouvant être non substitué ou porter un à trois des substituants suivants : nitro, cyano, halogéno, alkyle en C1-C4, halogénoalkyle en C1-C4, alcoxy en C1-C4, alkylthio en C1-C4, alcényloxy en C3-C6, alcynyloxy en C3-C6 et/ou NR^{a}R^{b} dans lequel
R^{a} représente l'hydrogène, un groupe alkyle en C1-C4, alcényle en C3-C6 ou alcynyle en C3-C6 et
R^{b} a l'une des significations indiquées pour R^{a} ou représente un groupe (alkyle en C1-C6)carbonyle ou benzoyle dont le cycle phényle peut lui-même porter un à trois des substituants suivants : nitro, cyano, halogéno, alkyle en C1-C4, halogénoalkyle en C1-C4, alcoxy en C1-C4 et/ou alkylthio en C1-C4 ;
R⁴ l'hydrogène ;
un groupe alkyle en C1-C10, halogénoalkyle en C1-C10, alcényle en C3-C10, halogénoalcényle en C3-C10, alcynyle en C3-C10, halogénoalcynyle en C3-C10, (alkyle en C1-C10)carbonyle ou (halogénoalkyle en C1-C10)carbonyle ;
un groupe benzoyle dont le cycle phényle est non substitué ou porte un à trois des substituants suivants : nitro, cyano, halogéno, alkyle en C1-C4, halogénoalkyle en C1-C4, alcoxy en C1-C4 et/ou alkylthio en C1-C4;
un groupe -S(=O)₂-R^{c} ; -P(=O) (OR^{d}) (OR^{e}) ou -SiR^{f}R^{g}R^{h} dans lequel
R^{c}, R^{d}, R^{e}, R^{g} et R^{h} représentent chacun, indépendamment les uns des autres, un groupe alkyle en C1-C10, halogénoalkyle en C1-C10 ou phényle pouvant lui-même porter un à trois des substituants suivants :
nitro, cyano, halogéno, alkyle en C1-C4, halogénoalkyle en C1-C4, alcoxy en C1-C4 et/ou alkylthio en C1-C4 ;
R³ ne pouvant représenter le groupe 2-(éthylthio)propyle lorsque R¹ représente un groupe éthyle, R² un groupe 3-chloro-2-propényle et R⁴ l'hydrogène,
ainsi que les sels des composés I et les esters d'acides carboxyliques en C1-C10 ou d'acides minéraux et des composés I convenant pour les applications agricoles.

2. Procédé de préparation des composés de formule la 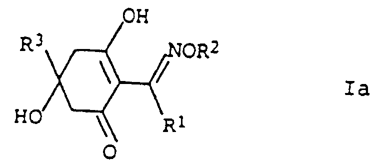 dans laquelle R¹, R² et R³ ont les significations indiquées dans la revendication 1, caractérisé par le fait que l'on cyclise une tricétone de formule II
(CH₃C=O)₂CH-CO-R¹ II
de manière connue en soi, dans un solvant organique inerte, en présence d'une base, à l'aide d'un halogénure d'acide de formule III
R³-CO-Hal III
dans laquelle Hal représente un atome d'halogène, en une cyclohexénone-cétone de formule IV 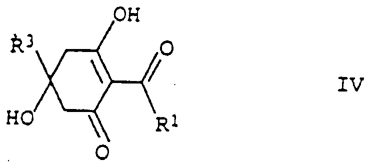 qu'on fait réagir ensuite de manière connue en soi, dans un solvant organique inerte, avec une hydroxylamine de formule V
H₂N-OR² V
ou avec un sel d'hydroxylammonium correspondant.

3. Procédé de préparation des composés de formule Ib 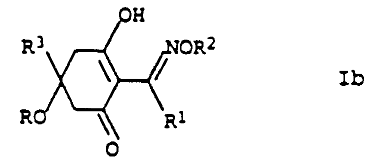 dans laquelle R¹, R² et R³ ont les significations indiquées dans la revendication 1 et R a l'une des significations indiquées dans la revendication 1 pour R⁴ à l'exception de l'hydrogène, caractérisé par le fait que l'on fait réagir un composé la selon la revendication 2, de manière connue en soi, dans un solvant organique inerte, avec un composé de formule VI
R-X VI
dans laquelle X représente un halogène.

4. Produits herbicides contenant une quantité herbicide efficace d'au moins un éther de cyclohexénonoxime de formule I selon la revendication 1 et des additifs inertes.

5. Procédé pour combattre les croissances de végétaux indésirables, caractérisé par le fait que l'on traite les végétaux et/ou leur habitat par une quantité efficace d'un éther de cyclohexénonoxime de formule I selon la revendication 1.

6. Utilisation des éthers de cyclohexénonoximes de formule I selon la revendication 1, pour la lutte contre les croissances de végétaux indésirables.

7. Cyclohexénone-cétones de formule IV selon la revendication 2.

8. Utilisation des cyclohexénone-cétones de formule IV selon la revendication 2 en tant que produits intermédiaires.
